# EUROPEAN PATENT SPECIFICATION

(11) **EP 4 115 858 B1**
(45) Date of publication and mention of the grant of the patent: **21.10.2026**
(21) Application number: 21779698.6
(22) Date of filing: 02.03.2021
(51) Int. Cl.: A61F 13/15, A61F 13/49, A61F 13/494, A61F 13/511, A61F 13/53, A61F 13/532, A61F 13/539

(54) **ABSORBENT ARTICLE AND METHOD FOR MANUFACTURING ABSORBENT ARTICLE**
SAUGFÄHIGER ARTIKEL UND VERFAHREN ZUR HERSTELLUNG DES SAUGFÄHIGEN ARTIKELS
ARTICLE ABSORBANT ET MÉTHODE DE FABRICATION D'UN ARTICLE ABSORBANT

(30) Priority: 31.03.2020 JP 2020064403
(43) Date of publication of application: 11.01.2023
(73) Proprietor: Unicharm Corporation, Shikokuchuo-shi, Ehime 799-0111 (JP)
(72) Inventor: MIYAMA, Takuya, Kanonji-shi, Kagawa 769-1602 (JP); UDA, Masashi, Kanonji-shi, Kagawa 769-1602 (JP)
(74) Representative: Dolleymores
(86) International application number: PCT/JP2021/007884
(87) International publication number: WO 2021/199846

(56) References cited:
- JP-A- 2007 117 727
- JP-A- 2011 152 209
- JP-A- 2017 202 238
- JP-A- 2019 097 947
- JP-A- 2019 216 957

## Description

### FIELD

The present invention relates to an absorbent article in which a protruded and recessed structure portion is located on a top sheet that comes into contact with the skin of a wearer and a method for manufacturing an absorbent article.

### BACKGROUND

Hitherto, in absorbent articles that absorb excrement such as urine or feces of wearers, an absorbent article in which a protruded and recessed structure portion is located on a top sheet that comes into contact with the skin of the wearer has been provided (for example, refer to Patent Literature 1). The top sheet of Patent Literature 1 has a large number of pleated portions that are parallel to each other, and is joined to a base sheet between the pleated portions. A portion of the top sheet joined to the base sheet constitutes a recessed portion, and an apex portion of the pleated portion constitutes a protruding portion. Each pleat portion extends in a front-rear direction and is close to an adjacent pleated portion in a width direction. The recessed portion of the top sheet is configured to take in highly viscous excrement such as soft feces. The highly viscous excrement is taken into the recessed portion, which makes it possible to separate the body and the highly viscous excrement.

### [CITATION LIST]

### [PATENT LITERATURE]

Patent Literature 1: Japanese Patent Application Publication No. 2002-165830
Patent Literature 2: Japanese Patent Application Publication No. 2011-152209

### SUMMARY

However, the top sheet of the absorbent article of Patent Literature 1 has the following problems. The moisture in excrement taken into a protruded and recessed structure portion of the top sheet is transferred toward the non-skin surface side. The thickness of the top sheet is larger than the thickness of the top sheet not having the protruded and recessed structure portion. Therefore, it sometimes takes time for transferring the moisture discharged onto the skin surface of the top sheet to the non-skin surface of the top sheet. In a case where a large amount of moisture is discharged, such as a case where urine and feces are discharged at one time, it takes a time to draw the bodily fluid toward the non-skin surface side with respect to the top sheet, and there is a possibility that excrement may be diffused through the top sheet. The excrement may be attached to the skin in a broad range to increase the load on the skin in some cases.

Accordingly, the present invention was achieved in order to solve the above-described problems, and an aspect of the present invention is to suppress the diffusion of excrement such as soft feces on a top sheet and to reduce the load on the skin.

An absorbent article according to one aspect having a front waist region, a rear waist region, and a crotch region that is positioned between the front waist region and the rear waist region, and having a front-rear direction that is directed from the front waist region toward the rear waist region, and a width direction that is orthogonal to the front-rear direction, comprises: an absorbent core that straddles the crotch region, extends toward at least any one of the front waist region and the rear waist region, and has at least a superabsorbent polymer; and a top sheet that is positioned on a skin facing surface side with respect to the absorbent core, and comes into contact with a wearer. In at least the rear waist region of the top sheet, a protruded and recessed structure portion having a top portion that protrudes toward the skin facing surface side, a bottom portion that protrudes toward a non-skin facing surface side, and a wall portion that connects the top portion and the bottom portion is located. The absorbent core has a low-basis-weight portion in which a basis weight of an absorbent material is lower than a basis weight of a surrounding region. A front end edge of the protruded and recessed structure portion is positioned on a rear side with respect to an article front-rear center, which is a center of the absorbent article in the front-rear direction. In a core arrangement region in which the absorbent core is arranged, an overlaid region in which the protruded and recessed structure portion and the low-basis-weight portion overlap, and a low-basis-weight region that overlaps the low-basis-weight portion in a plan view and in which the protruded and recessed structure position is not arrange.
At least a part of the low-basis-weight region is arranged on a front side with respect to the protruded and recessed structure portion. According to the present aspect, an absorbent article in which an overlaid region in which the protruded and recessed structure portion and the low-basis-weight portion overlap, and a front side region in which the
low-basis-weight portion is arranged on the front side with respect to the front end edge of the protruded and recessed structure portion are located can be manufactured.

A method for manufacturing an absorbent article according to an aspect is to manufacture an absorbent article having a front waist region, a rear waist region, and a crotch region that is positioned between the front waist region and the rear waist region, and having a front-rear direction that is directed from the front waist region toward the rear waist region, and a width direction that is orthogonal to the front-rear direction. A method for manufacturing an absorbent article includes a core molding step of molding an absorbent core having a low-basis-weight portion in which a basis weight of an absorbent material is lower than a basis weight of a surrounding region, a top sheet transport step of transporting a top sheet continuous body in which a top sheet that comes into contact with a wearer is continuous, a shaping step of forming a protruded and recessed structure portion having a top portion that protrudes toward a skin facing surface side, a bottom portion that protrudes toward a non-skin facing surface side, and a wall portion that connects the top portion and the bottom portion at least in the top sheet continuous body, and an overlaying step of overlaying the top sheet continuous body and the absorbent core in a manner such that the protruded and recessed structure portion and the low-basis-weight portion overlap in an overlaid region, and the low-basis-weight portion is arranged not to overlap the protruded and recessed structure portion in a low-basis-weight region. In the overlaying step, the low-basis-weight region is located on a front side with respect to the protruded and recessed structure portion.

### BRIEF DESCRIPTION OF DRAWINGS

FIG. 1 is an unfolded plan view of an absorbent article according to the present embodiment.
FIG. 2 is a cross-sectional view taken along a line A-A shown in FIG. 1.
FIG. 3 is a perspective view schematically showing a protruded and recessed structure portion.
FIG. 4 is a plan view for illustrating the positional relationship between the protruded and recessed structure portion and the low-basis-weight portion.
FIG. 5 is a diagram schematically showing an example of a method for manufacturing an absorbent article and an apparatus for manufacturing an absorbent article.

### DESCRIPTION OF EMBODIMENTS

### (1) Outline of embodiment

At least following matters will become clear with description of this specification and attached drawings.

An absorbent article according to one aspect having a front waist region, a rear waist region, and a crotch region that is positioned between the front waist region and the rear waist region, and having a front-rear direction that is directed from the front waist region toward the rear waist region, and a width direction that is orthogonal to the front-rear direction, comprises: an absorbent core that straddles the crotch region, extends toward at least any one of the front waist region and the rear waist region, and has at least a superabsorbent polymer; and a top sheet that is positioned on a skin facing surface side with respect to the absorbent core, and comes into contact with a wearer. In at least the rear waist region of the top sheet, a protruded and recessed structure portion having a top portion that protrudes toward the skin facing surface side, a bottom portion that protrudes toward a non-skin facing surface side, and a wall portion that connects the top portion and the bottom portion is located. The absorbent core has a low-basis-weight portion in which a basis weight of an absorbent material is lower than a basis weight of a surrounding region. A front end edge of the protruded and recessed structure portion is positioned on a rear side with respect to an article front-rear center, which is a center of the absorbent article in the front-rear direction. In a core arrangement region in which the absorbent core is arranged, an overlaid region in which the protruded and recessed structure portion and the low-basis-weight portion overlap, and a low-basis-weight region that does not overlap the protruded and recessed structure portion and overlaps the low-basis-weight portion are located. At least a part of the low-basis-weight region is arranged on a front side with respect to the protruded and recessed structure portion. According to the present aspect, since the protruded and recessed structure portion is located in the rear waist region, excrement such as soft feces can be retained in the bottom portion between the top portions. At least a part of the low-basis-weight region is arranged closer to a urethral orifice than the overlaid region. Therefore, at least a part of the absorbent core in the low-basis-weight region easily swells due to the absorption of moisture by the superabsorbent polymer, which makes it possible to suppress the transfer of excrement from the overlaid region side to the crotch side. Therefore, the diffusion of excrement such as soft feces on the top sheet can be suppressed and the load on the skin can be reduced. In addition, in the overlaid region, the protruded and recessed structure portion and the low-basis-weight portion are formed. The moisture in excrement taken into the protruded and recessed structure portion is transferred toward the non-skin surface side. The bodily fluid drawn into the non-skin surface side via the protruded and recessed structure portion can be diffused by the low-basis-weight portion. At this time, since the overlaid region is distant from the urethral orifice with respect to the low-basis-weight region, and urine is less likely to reach the overlaid region, the low-basis-weight portion of the low-basis-weight region can first swell and the transfer of excrement from the overlaid region side to the low-basis-weight region can be suppressed. Therefore, the diffusion of excrement such as soft feces on the top sheet can be suppressed and the load on the skin can be reduced.

According to a preferable aspect, the absorbent article further includes a core wrap that covers a skin surface and a non-skin surface of the absorbent core. The low-basis-weight portion is a slit in which the absorbent material is not substantially arranged. The core wrap positioned on a skin surface side of the absorbent core and the core wrap positioned on the non-skin surface of the absorbent core are joined in a region that overlaps the slit. According to the present aspect, the moisture discharged onto the top sheet is introduced into the low-basis-weight portion via the core wrap. At this time, the core wraps are joined to each other, and the moisture is easily introduced from the core wrap positioned on the skin surface side of the absorbent core to the core wrap positioned on the non-skin surface of the absorbent core. Further, since the core wraps are joined to each other, the core wraps easily come into close contact with the low-basis-weight portion, and the moisture is easily drawn into the low-basis-weight portion. Therefore, the bodily fluid can be prevented from continuously remaining on the top sheet and the load on the skin can be reduced.

According to a preferable aspect, in the core arrangement region, a protruded and recessed region that does not overlap the low-basis-weight portion and overlaps the protruded and recessed structure portion is located. At least a part of the protruded and recessed region is arranged on the rear side with respect to a rear end edge of the low-basis-weight portion. A front-rear length of the protruded and recessed region arranged on the rear side with respect to the rear end edge of the low-basis-weight portion is longer than a front-rear length of the overlaid region. According to the present aspect, when soft feces is discharged into the overlaid region, moisture is introduced from the top sheet to the low-basis-weight portion and diffused in the front-rear direction via the low-basis-weight portion. Since the front-rear length of the overlaid region is relatively short, the amount of moisture in soft feces introduced to the low-basis-weight portion can be suppressed and the wide range diffusion of the soft feces can be suppressed. Further, since the front-rear length of the protruded and recessed region arranged on the rear side with respect to the rear end edge of the low-basis-weight portion is relatively long, the drawing capacity of the soft feces in the protruded and recessed structure portion can be secured while suppressing the diffusion of the soft feces.

According to a preferable aspect, the absorbent article further includes an auxiliary sheet that comes into contact with a non-skin facing surface of the top sheet and has a higher density than the top sheet. The auxiliary sheet is arranged to straddle the overlaid region and the low-basis-weight region arranged on the front side with respect to the protruded and recessed structure portion. According to the present aspect, the density of the auxiliary sheet is relatively high, and the transfer of bodily fluid from the top sheet toward the auxiliary sheet can be facilitated. Therefore, the transfer of moisture from the top sheet side to the low-basis-weight portion can be further facilitated, the diffusion of excrement on the top sheet can be suppressed, and the load on the skin can be reduced.

According to a preferable aspect, the absorbent article further includes a core wrap that covers the absorbent core and comes into contact with a non-skin surface of the auxiliary sheet. The density of the core wrap is higher than the density of the auxiliary sheet. According to the present aspect, the density of the core wrap is relatively high, and the transfer of bodily fluid from the top sheet toward the auxiliary sheet and the transfer of the bodily fluid from the auxiliary sheet toward the core wrap can be facilitated. Therefore, the diffusion of excrement on the top sheet can be suppressed and the load on the skin can be reduced.

According to a preferable aspect, a pair of the low-basis-weight portions are located with a widthwise center of the absorbent article interposed therebetween. Each of the pair of low-basis-weight portions straddles the article front-rear center and is curved toward a widthwise outer side from the article front-rear center side toward a front-rear outer side. According to the present aspect, the low-basis-weight portion straddles the article front-rear center, and when the absorbent article is interposed between the legs of the wearer, the absorbent core is deformed with the low-basis-weight portion as a base point. The low-basis-weight portion is curved toward the widthwise outer side from the article front-rear center side toward the front-rear outer side, and a deformation base point due to the low-basis-weight portion easily extends along the inner side of the leg. When the low-basis-weight portion extends along the inner side of the leg and the absorbent core is deformed from the low-basis-weight portion as a base point, a cup shape in which a portion on the widthwise outer side with respect to the low-basis-weight portion for a region between the pair of low-basis-weight portions rises up can be embodied. The cup-shaped bottom portion (a region between the pair of low-basis-weight portions) makes it possible to diffuse a bodily fluid in the front-rear direction, and absorption of the bodily fluid by the entire absorbent core makes it possible to enhance the transferability of the bodily fluid from the top sheet toward the absorbent core. It should be noted that, in a modified example, a low-basis-weight portion 41 may have a linear shape that extends in the front-rear direction, or may have a linear shape that extends to be inclined with respect to the front-rear direction without being curved in a curved shape.

According to a preferable aspect, in the core arrangement region, a protruded and recessed region that does not overlap the low-basis-weight portion and overlaps the protruded and recessed structure portion, and a non-overlaid region that does not overlap the protruded and recessed structure portion and does not overlap the low-basis-weight portion are located. The non-overlaid region has a front non-overlaid region that extends toward a front side from the front end edge of the protruded and recessed structure portion. A basis weight of the superabsorbent polymer in the protruded and recessed region is higher than a basis weight of the superabsorbent polymer in the front non-overlaid region. According to the present aspect, the protruded and recessed region overlaps the protruded and recessed structure portion, and is a region in which soft feces is discharged. The front non-overlaid region is positioned on the front side with respect to the protruded and recessed region, and is a region in which urine is more easily discharged than the protruded and recessed region. Compared with urine, the moisture in soft feces is less likely to be transferred to the superabsorbent polymer and is less likely to be absorbed by the superabsorbent polymer. When the basis weight of the superabsorbent polymer is increased in a region in which soft feces which is less likely to be absorbed by the superabsorbent polymer is discharged (protruded and recessed region), the absorbability for the moisture of the soft feces by the superabsorbent polymer can be enhanced.

According to a preferable aspect, the absorbent core has a surrounding portion that surrounds the low-basis-weight portion in a plan view. A basis weight of the superabsorbent polymer in the surrounding portion is higher than a basis weight of the superabsorbent polymer in a region on an outer side with respect to the low-basis-weight portion and the surrounding portion. According to the present aspect, since the basis weight of the superabsorbent polymer in the surrounding portion that surrounds the low-basis-weight portion is high, the moisture drawn into the low-basis-weight portion can be absorbed by the superabsorbent polymer. The low-basis-weight portion more easily swells, and an effect of suppressing the transfer of excrement from the overlaid region side to the crotch region side can be more easily obtained.

According to a preferable aspect, the absorbent article further includes a rising leak-proof gather having a contraction portion that contracts by contraction of a leak-proof elastic member, and a rear base point portion that serves as a rising fulcrum of the contraction portion on the rear side with respect to the contraction portion. A front end edge of the rear base point portion is positioned on the rear side with respect to a rear end edge of the protruded and recessed structure portion. The contraction portion of the leak-proof gather rises up from the rear base point portion as a fulcrum, and a pocket covered with the leak-proof gather is formed on the front side with respect to the rear base point portion. Since the rear end edge of the protruded and recessed structure portion is positioned in the pocket, even when soft feces is diffused into the protruded and recessed structure portion, the soft feces can be stored in the pocket. Therefore, soft feces can be prevented from being attached to the skin and the load on the skin can be reduced.

According to a preferable aspect, the absorbent core overlaps the front end edge of the rear base point portion and extends toward the rear side with respect to the front end edge of the rear base point portion According to the present aspect, since the absorbent core overlaps the front end edge of the rear base point portion and the absorbent core extends toward the rear side with respect to the front end edge of the rear base point portion, the rear base point portion is stabilized by the stiffness of the absorbent core. The leak-proof gather easily rises up, and a pocket that stores soft feces can be easily formed. Therefore, the soft feces is more easily stored in the pocket, soft feces can be prevented from being attached to the skin, and the load on the skin can be reduced.

According to a preferable aspect, the absorbent article further includes a waist gather that stretches and contracts in the width direction in the rear waist region. The waist gather is arranged on the rear side with respect to a rear end edge of the absorbent core. According to the present aspect, a region on the rear side with respect to the rear end edge of the absorbent core easily comes into close contact with the body with the waist gather. A region on the front side with respect to the waist gather is less likely to come into close contact with the region that overlaps the waist gather, and a pocket that bulges toward the body with respect to the waist gather can be formed. When the pocket having the front end edge of the waist gather as a base point is formed on the front side with respect to the waist gather, and the pocket having the front end edge of the rear base point portion as a base point is formed on the front side with respect to the rear end edge of the absorbent core, even in case where soft feces is diffused into the protruded and recessed structure portion, the soft feces can be stored in the pockets. Therefore, the soft feces is more easily stored in the pocket, soft feces can be prevented from being attached to the skin, and the load on the skin can be reduced.

A method for manufacturing an absorbent article according to an aspect is to manufacture an absorbent article having a front waist region, a rear waist region, and a crotch region that is positioned between the front waist region and the rear waist region, and having a front-rear direction that is directed from the front waist region toward the rear waist region, and a width direction that is orthogonal to the front-rear direction. A method for manufacturing an absorbent article includes a core molding step of molding an absorbent core having a low-basis-weight portion in which a basis weight of an absorbent material is lower than a basis weight of a surrounding region, a top sheet transport step of transporting a top sheet continuous body in which a top sheet that comes into contact with a wearer is continuous, a shaping step of forming a protruded and recessed structure portion having a top portion that protrudes toward a skin facing surface side, a bottom portion that protrudes toward a non-skin facing surface side, and a wall portion that connects the top portion and the bottom portion at least in the top sheet continuous body, and an overlaying step of overlaying the top sheet continuous body and the absorbent core in a manner such that the protruded and recessed structure portion and the low-basis-weight portion overlap in an overlaid region, and the low-basis-weight portion is arranged not to overlap the protruded and recessed structure portion in a low-basis-weight region. In the overlaying step, the low-basis-weight region is located on a front side with respect to the protruded and recessed structure portion.

According to a preferable aspect, the method further includes a joining step of joining a non-skin surface of the protruded and recessed structure portion and a non-skin side member that comes into contact with the non-skin surface of the protruded and recessed structure portion in a joining region. In the joining step, at least the joining region is located in a region in which the low-basis-weight portion is not arranged. According to the present aspect, the portion in which the protruded and recessed structure portion is formed has a shape that protrudes and is recessed in the thickness direction, which makes it difficult to obtain an area in contact with the non-skin surface member, compared with a smooth portion. In addition, in the portion in which the low-basis-weight portion is arranged, the thickness of the absorbent core is small compared with the surrounding portion, and it is difficult to obtain an area in contact with portion in which the protruded and recessed structure portion is formed. When the joining region is located only in the portion in which the low-basis-weight portion is arranged and the portion in which the protruded and recessed structure portion is formed, it is difficult to obtain the joining force of the joining region in some cases. However, when at least the joining region is located in the region in which the low-basis-weight portion is not arranged, the joining force of the joining region can be obtained.

### (2) Absorbent article according to an embodiment

Next, embodiments of an absorbent article 10 according to the present invention will be described with reference to the accompanying drawings. The absorbent article according to the present invention absorbs the bodily fluid of a wearer. A target wearer can be exemplified by an infant and an elderly person. The absorbent article may be a disposable diaper or an incontinence pad that absorbs urine, a sanitary napkin that absorbs menstrual blood, or a panty liner that absorbs the vaginal discharge. The disposable diaper may be an underpants-shaped diaper or a tape-type diaper. The absorbent article according to the embodiment is a tape-type disposable diaper. Further, in the following description of the drawings, identical or similar portions are denoted by identical or similar reference signs. Here, the drawings are schematic views, and attention needs to be paid to the fact that the ratios between individual dimensions and the like are different from actual ones. Therefore, specific dimensions and the like need to be determined with reference to the following description. In addition, there may be a portion where the relationship or ratio between dimensions does not match between drawings.

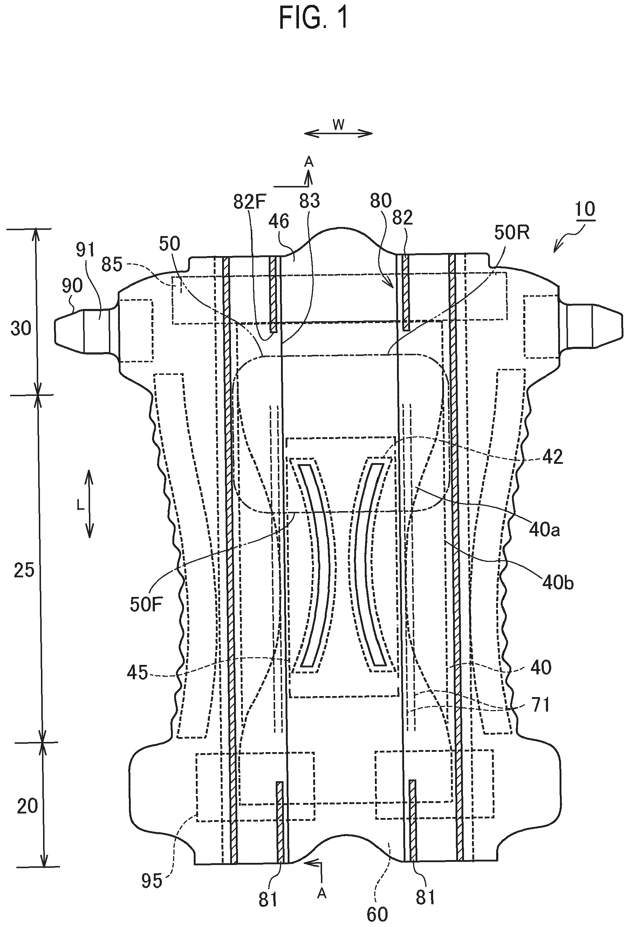

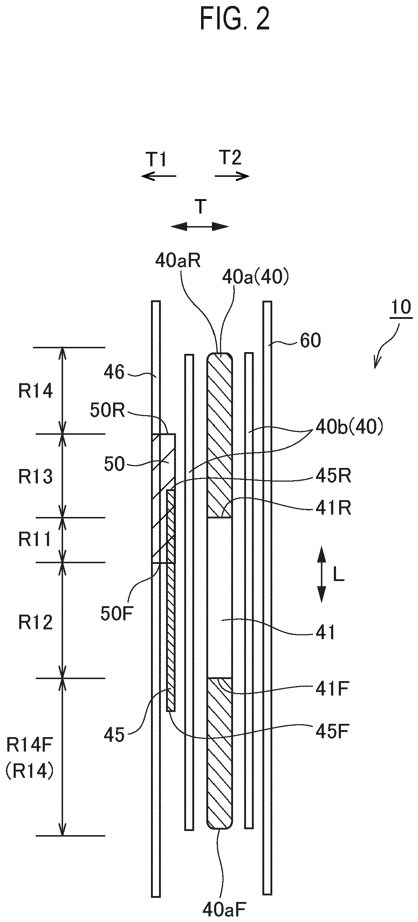

FIG. 1 is a schematic plan view of the absorbent article 10 according to the embodiment as seen from a skin facing surface side T1 in an unfolded state. The schematic plan view shown in FIG. 1 shows a stretched state in which the absorbent article 10 is stretched to a state in which no wrinkles are formed. Fig. 2 is a schematic cross-sectional view taken along a line A-A shown in Fig. 1. In the schematic cross-sectional view, for convenience of description, each member may be spaced apart in the thickness direction T. However, in an actual product, each member is in contact with each other in the thickness direction T. The absorbent article 10 includes a front waist region 20, a crotch region 25, and a rear waist region 30. The front waist region 20 is a portion that comes into contact with the front waist portion (abdomen portion) of a wearer. Further, the rear waist region 30 is a portion that comes into contact with the rear waist portion (rear portion) of the wearer. The crotch region 25 is positioned between the front waist region 20 and the rear waist region 30. In the present embodiment, it should be noted that a direction from the front waist region 20 toward the rear waist region 30 will be referred to as a front-rear direction L, a direction orthogonal to the front-rear direction L will be referred to as a width direction W, and a direction that extends to the skin facing surface side T1 and a non-skin facing surface side T2 of the wearer will be referred to as a thickness direction T.

The absorbent article 10 has an absorbent body 40. The absorbent body 40 may include an absorbent core 40a and a core wrap 40b. The absorbent core 40a includes an absorbent material such as pulp and a superabsorbent polymer. The absorbent core 40a may include at least a superabsorbent polymer. The absorbent core 40a straddles the crotch region 25 and extends toward at least any one of the front waist region 20 and the rear waist region 30. The absorbent core 40a of the present embodiment is arranged to straddle the front waist region 20, the crotch region 25, and the rear waist region 30. The absorbent core 40a has the low-basis-weight portion 41 in which the basis weight of the absorbent material is lower than the basis weight of the surrounding region. The low-basis-weight portion 41 is a portion in which the weight (g/m³) of the absorbent material per unit area is lower than the weight of the surrounding region. The low-basis-weight portion may be a portion in which an absorbent material is arranged or may be a slit having a basis weight of substantially 0 (having a basis weight of 0 in design). In the low-basis-weight portion formed of the slit, the absorbent material that has fed from the surrounding region may be slightly arranged. The front-rear length of the low-basis-weight portion 41 may be shorter than the front-rear length of the absorbent core 40a. A pair of low-basis-weight portions 41 may be located with an article width center 10 WC, which is the center of the absorbent article in the width direction W, interposed therebetween. In such an aspect, the low-basis-weight portions 41 may not be arranged at the article width center 10WC but may be spaced apart from the article width center 10WC. The pair of low-basis-weight portions 41 may be linearly symmetrical with respect to the article width center 10WC as a center. The core wrap 40b covers the absorbent core 40a. At least a part of the core wrap 40b comes into contact with the non-skin surface of an auxiliary sheet 45 described later. The core wrap 40b may be arranged so as to cover the skin surface of the absorbent core 40a and the non-skin surface of the absorbent core 40a.

The absorbent article 10 has a top sheet 46. The top sheet 46 is positioned on the skin facing surface side T1 with respect to the absorbent core 40a and comes into contact with the wearer. The top sheet 46 has liquid permeability. The top sheet 46 constitutes a skin contact surface of the absorbent article 10. The top sheet 46 is arranged to straddle the front waist region 20, the crotch region 25, and the rear waist region 30. The top sheet 46 has fibers, and specifically, can be formed of a nonwoven fabric. At least in the rear waist region 30 of the top sheet 46, a protruded and recessed structure portion 50 is located. The protruded and recessed structure portion 50 will be described in detail later. The absorbent article 10 may have a side sheet 70 that covers the outer side portion of the top sheet 46. The side sheet 70 is arranged on the skin facing surface side of the top sheet 46. Therefore, a region of the top sheet 46 that is not covered with the side sheet 70 comes into contact with the skin of the wearer. The side sheet 70 constitutes a leak-proof gather 80 described below. On the non-skin facing surface side T2 of the absorbent body 40, a liquid-impermeable back sheet 60 is provided. On the non-skin facing surface side T2 of the back sheet 60, an exterior sheet (not shown) formed of a nonwoven fabric may be arranged.

The absorbent article 10 may have the auxiliary sheet 45 that comes into contact with the non-skin facing surface side T2 of the top sheet 46. The auxiliary sheet 45 is arranged between the top sheet 46 and the absorbent body 40. The auxiliary sheet 45 of the present embodiment is arranged between the top sheet 46 and the core wrap 40b. The area of the auxiliary sheet 45 may be smaller than the area of the top sheet 46. More specifically, the length of the auxiliary sheet 45 in front-rear direction L may be shorter than the length of the top sheet 46 in the front-rear direction L and may be shorter than the length of the absorbent core 40a in the front-rear direction L The length of the auxiliary sheet 45 in the width direction W may be shorter than the length of the top sheet 46 in the width direction W and may be shorter than the length of the absorbent core 40a in the width direction W. A region in which the top sheet 46 and the auxiliary sheet 45 are overlaid on each other, and a region in which the top sheet 46 is arranged and the auxiliary sheet 45 is not arranged (the top sheet 46 and the auxiliary sheet 45 are not overlaid on each other) may be located.

A pair of leak-proof gathers 80 are arranged on two widthwise outer sides with respect to widthwise center of the absorbent article 10. The leak-proof gather 80 is a rising gather capable of rising toward the wearer. The leak-proof gather 80 is configured to include a side sheet 70 and a leak-proof elastic member 71. The leak-proof elastic member 71 is joined to the inner side portion of the side sheet 70, and stretches and contracts in the front-rear direction L. The pair of leak-proof gathers 80 are substantially linearly symmetrical with respect to the widthwise center of the absorbent article as a symmetry axis. The leak-proof gather 80 may include a contraction portion 83 that contracts due to the contraction of the leak-proof elastic member 71, a rear base point portion 82 that serves as a rising fulcrum of the contraction portion 83 on the rear side with respect to the contraction portion 83, and a front base point portion 81 that serves as a rising fulcrum of the contraction portion 83 on the front side with respect to the contraction portion 83. The side sheet 70 is joined to the top sheet 46 at the front base point portion 81 and the rear base point portion 82, and is not joined to the top sheet 46 at the contraction portion 83. Therefore, the contraction portion 83 rises up toward the wearer with the front base point portion 81 and the rear base point portion 82 as the base points due to the contraction of the leak-proof elastic member 71. The leak-proof gather 80 forms a wall that rises up toward the skin facing surface side T1 at the outer side edge of the absorbent body 40 to prevent lateral leakage of excrement.

The absorbent article 10 includes a pair of fastening tapes 90. The fastening tape 90 extends toward the outer side in the width direction W with respect to the side sheet 70 in the rear waist region 30. The fastening tape 90 has a fastening portion 91 that is fastened to a target portion 95 of the front waist region 20. The fastening portion 91 is, for example, a portion located with an engaging hook. The fastening tapes 90 are fastened to the target portions 95 of the front waist region 20 to retain the absorbent article 10 to the body of the wearer. The fastening tapes 90 are attached to the side sheets 70. The target portion 95 is located on the non-skin facing side surface of the exterior sheet in the front waist region 20. The target portion 95 is configured to be caught by the engaging hook of the fastening tape 90, and functions as a loop of the hook-and-loop fastening system. The absorbent article 10 has a waist gather 85 that stretches and contracts in the width direction. The waist gather 85 is arranged in the rear waist region 30. The waist gather 85 is arranged between the pair of fastening tapes 90 in the width direction, and contracts in the width direction between the fastening tapes 90.

### (3) Configuration of Protruded and Recessed Structure Portion

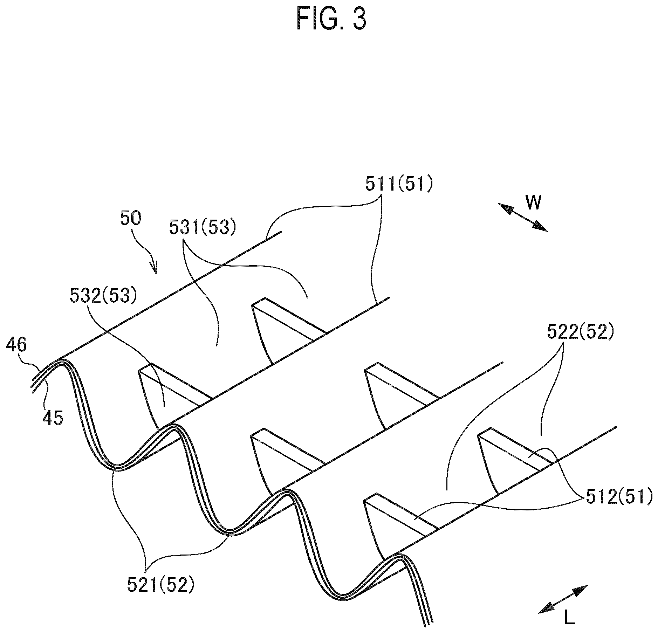

Next, the configuration of the protruded and recessed structure portion 50 will be described in detail. FIG. 3 is an enlarged plan view of the protruded and recessed structure portion 50. The protruded and recessed structure portion 50 includes top portions 51 that protrude toward the skin facing surface side T1, bottom portions 52 that protrude toward the non-skin facing surface side T2, and wall portions 53 that connect the top portions 51 and the bottom portions 52. In FIGS. 1 and 2, the detailed structure of the protruded and recessed structure portion 50 is omitted. The top portion 51 protrudes toward the skin facing surface side T1 with respect to the surrounding region. The bottom portion 52 protrudes toward the non-skin facing surface side T2 with respect to the surrounding region. The wall portion 53 is a portion that connects the top portion 51 and the bottom portion 52, and may extend in at least the thickness direction T. The top portion 51 is a portion including a point positioned on the most skin facing surface side T1, the bottom portion 52 is a portion including a point positioned on the most non-skin facing surface side T2, and the wall portion 53 is a portion between the top portion 51 and the bottom portion 52.

The protruded and recessed structure portion 50 of the present embodiment includes first top portions 511, first bottom portions 521, and first wall portions 531, each of which extends in the front-rear direction L, and second top portions 512, second bottom portions 522, and second wall portions 532, each of which extends in the width direction W. The first top portions 511, the first bottom portions 521, and the first wall portions 531 are arranged with spaces in the width direction W, and are arranged to be adjacent to each other in a cross section along the width direction W. Further, the second top portions 512, the second bottom portions 522, and the second wall portions 532 are arranged with spaces in the front-rear direction L, and are arranged to be adjacent to each other in a cross section along the front-rear direction L. As described above, in the aspect including the protruded and recessed structure portion 50 that extends in the width direction W and the protruded and recessed structure portion 50 that extends in the front-rear direction L, the diffusion of feces in the width direction W can be suppressed and the diffusion of feces in the front-rear direction L can also be suppressed. In addition, in an aspect having the protruded and recessed structure portion 50 that extends in the width direction W and the protruded and recessed structure portion 50 that extends in the front-rear direction L, the top portion and the bottom portion can be set in each of cross sections of the top portion and the bottom portion (that extend in the front-rear direction L) in a cross section along the width direction W, and the top portion and the bottom portion (that extend in the width direction W) in a cross section along the front-rear direction L. Further, the protruded and recessed structure portion 50 may have any form as long as the structure portion has a top portion, a bottom portion, and a wall portion, and the configuration is not limited to the present embodiment. A well-known structure can be employed. For example, a protruded and recessed structure portion 50 described in Japanese Patent No. 6087462 can be employed.

The protruded and recessed structure portion 50 is formed in at least the top sheet 46. That is, the protruded and recessed structure portion 50 is formed on the skin facing surface (the surface that comes into contact with the skin of the wearer) of the absorbent article. It should be noted that the protruded and recessed structure portion 50 may be integrally formed with the top sheet 46 and the auxiliary sheet 45 in a region in which the top sheet 46 and the auxiliary sheet 45 are arranged. In the aspect in which the protruded and recessed structure portion is integrally formed with the top sheet 46 and the auxiliary sheet 45, the protruded and recessed structure portion (for example, protruding portions) of the top sheet 46 and the protruded and recessed structure portion (for example, protruding portions) of the auxiliary sheet 45 are not separately formed, both are formed together, and the protruding portions of the auxiliary sheet 45 are arranged along the protruding portions of the top sheet 46. In a method for manufacturing an absorbent article described below, the protruded and recessed structure portion 50 subjected to the shaping process in a state in which the top sheet 46 and the auxiliary sheet 45 are overlaid on each other becomes a protruded and recessed structure portion 50 which is integrally formed with the top sheet 46 and the auxiliary sheet 45.

The protruded and recessed structure portion 50 is arranged in at least the rear waist region 30. Therefore, feces are mainly discharged onto the protruded and recessed structure portion 50. When feces are discharged onto the protruded and recessed structure portion 50, the feces are stored in the bottom portions 52 between the top portions 51. By drawing the feces into the protruded and recessed structure portion 50, discomfort caused by the feces continuing to come into contact with the skin of the wearer can be reduced. Further, in the aspect in which the protruded and recessed structure portion 50 is integrally formed with the top sheet 46 and the auxiliary sheet 45, the protruded and recessed structure portion 50 of the top sheet 46 overlaps the protruded and recessed structure portion 50 of the auxiliary sheet 45 and extends along the protruded and recessed structure portion 50 of the auxiliary sheet 45. Therefore, the moisture in excrement on the top sheet 46 is easily transferred to the auxiliary sheet 45. Therefore, the transfer of the bodily fluid from the top sheet 46 to the auxiliary sheet 45 can be facilitated while introducing the bodily fluid toward the non-skin facing surface side T2.

The density of the bottom portion 52 may be higher than the density of the wall portion 53. It is sufficient that the density of at least a part of the bottom portion 52 is higher than the density of the wall portion 53, and the bottom portion 52 may have a portion having the same density as the wall portion 53. Since the density of the bottom portion 52 is higher than the density of the wall portion 53, the bodily fluid discharged onto the top sheet 46 is easily introduced into the bottom portion 52. The transfer of the bodily fluid from the top sheet 46 to the auxiliary sheet 45 can be facilitated while introducing the bodily fluid toward the non-skin facing surface side. It should be noted that, a portion having a high density and a portion having a low density can be determined by the following method. The density can be determined by enlarging a target portion of the sheet (for example, a size of 1 mm × 1 mm) by a digital microscope, an electron microscope, or the like to approximately 100 times, and comparing the number of fibers present on the sheet surface in a unit area. Preferably, 30 positions are checked in each of the regions to be measured, and, from the tendency of the number comparison, a target portion where the number of fibers is likely to be large is defined as a region where the fiber density is high.

Further, the density of the top portion 51 may be higher than the density of the wall portion 53. Since the density of the top portion 51 and the density of the bottom portion 52 are higher than the density of the wall portion 53, the bodily fluid discharged onto the top sheet 46 is easily introduced to the top portion 51 and the bottom portion 52. When the bodily fluid is introduced into the bottom portion 52, the bodily fluid can be transferred to the auxiliary sheet 45 while introducing the bodily fluid into the side away from the body. This makes it possible to suppress the problem of the bodily fluid continuing to come into contact with the skin. Further, the bodily fluid introduced to the top portion 51 and introduced from the top portion 51 toward the auxiliary sheet 45 is introduced into the space on the back side of the bulge of the top portion 51. When the bodily fluid is drawn into the space on the back side of the top portion 51, the bodily fluid is introduced into the side away from the body, which makes it possible to suppress the problem of the bodily fluid continuing to come into contact with the skin.

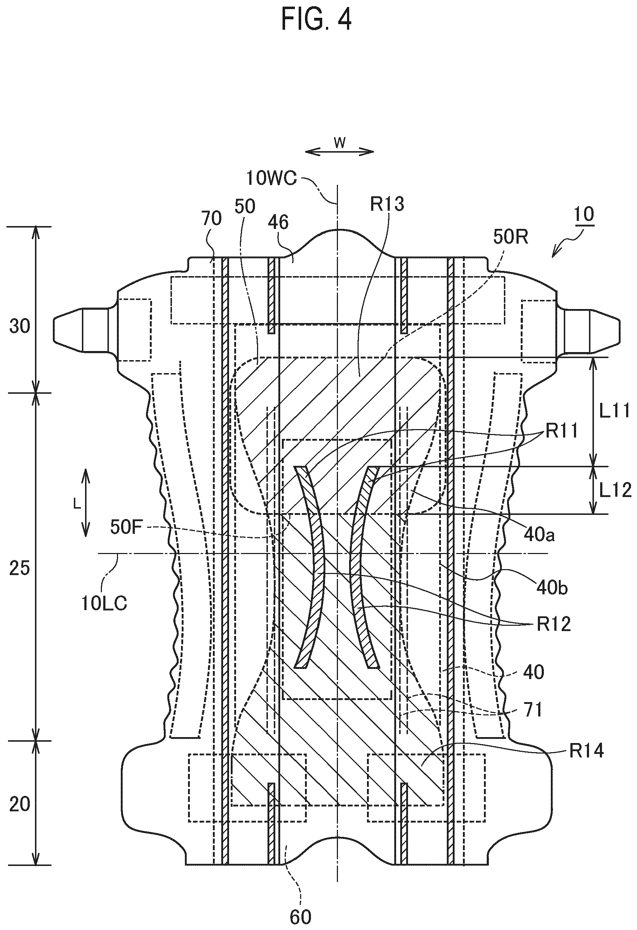

Next, with reference to FIG. 4 and the like, the positional relationship between the protruded and recessed structure portion 50 and the low-basis-weight portion 41 will be described. FIG. 4 is a plan view of the absorbent article shown in FIG. 1 and is a diagram showing the positional relationship between the protruded and recessed structure portion 50 and the low-basis-weight portion 41. The protruded and recessed structure portion 50 are formed on a part of the top sheet 46 in a plan view. A front end edge 50F of the protruded and recessed structure portion 50 is positioned on the rear side with respect to an article front-rear center 10LC, which is a center of the absorbent article in the front-rear direction, and is positioned on the rear side with respect to a front end edge 41F of the low-basis-weight portion 41. A rear end edge 50R of the protruded and recessed structure portion 50 is positioned on the front side with respect to the rear end edge of the absorbent core 40a, and is positioned on the rear side with respect to a rear end edge 41R of the low-basis-weight portion 41. The inner side edge (the widthwise inner side end edge) of the low-basis-weight portion 41 may be positioned on the widthwise outer side with respect to the article width center 10WC, the outer side edge (the widthwise outer side end edge) of the low-basis-weight portion 41 may be positioned on the widthwise inner side with respect to the outer side edge of the absorbent core 40a, and may be positioned on the widthwise inner side with respect to the outer side edge of the protruded and recessed structure portion 50.

In the core arrangement region in which the absorbent core 40a is arranged, at least an overlaid region R11 in which the protruded and recessed structure portion 50 and the low-basis-weight portion 41 overlap, and a low-basis-weight region R12 that does not overlap the protruded and recessed structure portion 50 and overlaps the low-basis-weight portion 41 may be located. The overlaid region R11 is a region in which the protruded and recessed structure portion 50 and the low-basis-weight portion 41 are arranged. The front end edge of the overlaid region R11 matches the front end edge 50F of the protruded and recessed structure portion 50 and is positioned on the rear side with respect to the front end edge of the low-basis-weight portion 41. The rear end edge of the overlaid region R11 matches the rear end edge of the low-basis-weight portion 41 and is positioned on the front side with respect to the rear end edge 50R of the protruded and recessed structure portion 50. The outer side edge of the overlaid region R11 matches the outer side edge of the low-basis-weight portion 41, and is positioned on the widthwise inner side with respect to the outer side edge of the absorbent core 40a and on the widthwise inner side with respect to the outer side edge of the protruded and recessed structure portion 50. The low-basis-weight region R12 is a region in which the protruded and recessed structure portion 50 is not arranged and the low-basis-weight portion 41 is arranged. The low-basis-weight region R12 is a region that overlaps the low-basis-weight portion 41 in a plan view and in which the protruded and recessed structure portion 50 is not arranged. At least a part of the low-basis-weight region R12 is arranged on the front side with respect to the protruded and recessed structure portion 50. The rear end edge of the low-basis-weight region R12 matches the front end edge 50F of the protruded and recessed structure portion 50, and the front end edge of the low-basis-weight region R12 matches the front end edge 41F of the low-basis-weight portion 41. That is, the low-basis-weight region R12 extends from the front end edge 50F of the protruded and recessed structure portion 50 toward the front side. At least a part of the low-basis-weight region R12 is arranged closer to the urethral orifice than the overlaid region R11. Therefore, at least a part of the absorbent core 40a of the low-basis-weight region R12 easily swells due to the absorption of moisture by the superabsorbent polymer, which makes it possible to suppress the transfer of excrement from the overlaid region R11 side to the crotch side. Therefore, the diffusion of excrement such as soft feces on the top sheet 46 can be suppressed and the load on the skin can be reduced. In addition, in the overlaid region R11, the protruded and recessed structure portion 50 and the low-basis-weight portion 41 are formed. The moisture in excrement taken into the protruded and recessed structure portion 50 is transferred toward the non-skin surface side. This makes it possible for the low-basis-weight portions 41 to diffuse the bodily fluid that has been drawn to the non-skin surface side via the protruded and recessed structure portions 50. At this time, since the overlaid region R11 is distant from the urethral orifice with respect to the low-basis-weight region R12, and urine is less likely to reach the overlaid region, the low-basis-weight portion 41 of the low-basis-weight region R12 can first swell and the transfer of excrement from the overlaid region R11 side to the low-basis-weight region R12 can be suppressed. Therefore, the diffusion of excrement such as soft feces on the top sheet 46 can be suppressed and the load on the skin can be reduced.

In the core arrangement region, a protruded and recessed region R13 that does not overlap the low-basis-weight portion 41 and overlaps the protruded and recessed structure portion 50 may be located. The protruded and recessed region R13 is a region in which the protruded and recessed structure portion 50 is arranged and the low-basis-weight portion 41 is not arranged. The front end edge of the protruded and recessed region R13 matches the front end edge 50F of the protruded and recessed structure portion 50 and is positioned on the rear side with respect to the front end edge of the low-basis-weight portion 41. The rear end edge of the protruded and recessed region R13 matches the rear end edge 50R of the protruded and recessed structure portion 50 and is positioned on the rear side with respect to the rear end edge 41R of the low-basis-weight portion 41. The outer side edge of the protruded and recessed region R13 matches the outer side edge of the protruded and recessed structure portion 50 and is positioned on the widthwise outer side with respect to the outer side edge of the low-basis-weight portion 41. The protruded and recessed region R13 is a region in which the protruded and recessed structure portion 50 is arranged and the low-basis-weight portion 41 is not arranged in a plan view. Therefore, the protruded and recessed region R13 is located in a region between the pair of low-basis-weight portions 41 and in a region on the widthwise outer side with respect to each of the low-basis-weight portions. At least a part of the protruded and recessed region R13 is arranged on the rear side with respect to the rear end edge 41R of the low-basis-weight portion 41. A front-rear length L11 of the protruded and recessed region R13 arranged on the rear side with respect to the rear end edge 41R of the low-basis-weight portion 41 may be longer than a front-rear length L12 of the overlaid region R11. When soft feces is discharged to the overlaid region R11, the moisture in the soft feces is introduced from the top sheet 46 to the low-basis-weight portion 41 and diffused in the front-rear direction via the low-basis-weight portion 41. Since the front-rear length of the overlaid region R11 is relatively short, the amount of moisture in soft feces introduced to the low-basis-weight portion 41 can be suppressed and the wide range diffusion of the soft feces can be suppressed. Further, since the front-rear length of the protruded and recessed region R13 arranged on the rear side with respect to the rear end edge 41R of the low-basis-weight portion 41 is relatively long, the drawing capacity of the soft feces in the protruded and recessed structure portion 50 can be secured while suppressing the diffusion of the soft feces. By drawing the feces into the protruded and recessed structure portion 50, discomfort caused by the feces continuing to come into contact with the skin of the wearer can be reduced.

In the core arrangement region, a non-overlaid region R14 that does not overlap the protruded and recessed structure portion 50 and does not overlap the low-basis-weight portion 41 may be located. The non-overlaid region R14 is a region in which the protruded and recessed structure portion 50 is not arranged and the low-basis-weight portion 41 is not arranged. The non-overlaid region R14 is located at least in a region on the front side with respect to the low-basis-weight portion 41 and a region on the widthwise outer side with respect to the protruded and recessed structure portion 50. The non-overlaid region R14 is located in a region between the pair of low-basis-weight portions and in a region on the widthwise outer side with respect to each of the low-basis-weight portions 41 on the front side with respect to the protruded and recessed structure portion 50. As shown in FIG. 2, the non-overlaid region R14 may have a front non-overlaid region R14F that extends from the front end edge 50F of the protruded and recessed structure portion 50 toward the front side. The front non-overlaid region R14F is a region in which the low-basis-weight portion 41 is not arranged in the region that extends toward the front side from the front end edge 50F of the protruded and recessed structure portion 50. The protruded and recessed region R13 overlaps the protruded and recessed structure portion 50, and is a region in which soft feces is discharged. The front non-overlaid region R14F is a region that is positioned on the front side with respect to the protruded and recessed region R13, and in which urine is more easily discharged than the protruded and recessed region R13. Compared with urine, the moisture in soft feces is less likely to be transferred to the superabsorbent polymer and is less likely to be absorbed by the superabsorbent polymer. When the basis weight of the superabsorbent polymer is increased in a region in which soft feces which is less likely to be absorbed by the superabsorbent polymer is discharged (protruded and recessed region), the absorbability for the moisture of the soft feces by the superabsorbent polymer can be enhanced.

The core wrap 40b positioned on the skin surface side of the absorbent core 40a and the core wrap 40b positioned on the non-skin surface of the absorbent core 40a may be joined in a region that overlaps the slit constituting the low-basis-weight portion 41. The moisture discharged onto the top sheet 46 is introduced into the low-basis-weight portion 41 via the core wrap 40b. At this time, the core wraps 40b are joined to each other, and the moisture is easily introduced from the core wrap 40b positioned on the skin surface side of the absorbent core 40a to the core wrap 40b positioned on the non-skin surface of the absorbent core 40a. Further, since the core wraps 40b are joined to each other, the core wraps 40b easily come into close contact with the low-basis-weight portion 41, and the moisture is easily drawn into the low-basis-weight portion 41. Therefore, the bodily fluid can be prevented from continuously remaining on the top sheet 46 and the load on the skin can be reduced. Further, moisture is easily introduced into the core wrap 40b positioned on the non-skin surface of the absorbent core 40a, and excrement can be brought closer to the non-skin surface side of the absorbent article. Therefore, the wearing assistant can grasp that there is excrement from the non-skin surface side of the absorbent article, the change of the absorbent article can be promoted, and the excrement can be prevented from being continuously attached to the skin. It should be noted that the core wraps may be joined to each other by thermal welding or ultrasonic welding. According to the above-described configuration, compared with a configuration in which the top sheet continuous body and the auxiliary sheet are joined via a hotmelt adhesive, a decrease in the drawability of bodily fluid due to the adhesive can be suppressed. Therefore, the moisture can be drawn into the low-basis-weight portion 41 via the core wrap while suppressing a decrease in the drawability of the bodily fluid.

The density of the auxiliary sheet 45 may be higher than the density of the top sheet 46. The auxiliary sheet 45 may be arranged to straddle the overlaid region R11 and the low-basis-weight region R12 arranged on the front side with respect to the protruded and recessed structure portion 50. The relatively high density of the auxiliary sheet 45 makes it possible to facilitate the transfer of bodily fluid from the top sheet 46 side toward the auxiliary sheet 45. The transfer of moisture from the top sheet 46 side to the low-basis-weight portion 41 can be further facilitated, the diffusion of excrement on the top sheet can be suppressed, and the load on the skin can be reduced. Suitably, the auxiliary sheet 45 may cover the entire region of the low-basis-weight portion 41. That is, the rear end edge of the auxiliary sheet 45 may be positioned on the rear side with respect to the rear end edge 41R of the low-basis-weight portion 41, a front end edge 45F of the auxiliary sheet may be positioned on the front side with respect to the front end edge 41F of the low-basis-weight portion 41, and the outer side edge of the auxiliary sheet may be positioned on the widthwise outer side with respect to the outer side edge of the low-basis-weight portion 41. The transfer of moisture from the top sheet side to the low-basis-weight portion 41 can be further facilitated, the diffusion of excrement on the top sheet can be suppressed, and the load on the skin can be further reduced. It should be noted that a portion in which the density is high and a portion in which the density is low in the sheet can be determined by a difference in fiber diameter. In a case where the fiber diameter is small, it can be determined that the interfiber distance is short and the density is high. Further, the density of the core wrap 40b may be higher than the density of the auxiliary sheet 45. The relatively high density of the core wrap makes it possible to facilitate the transfer of bodily fluid from the top sheet 46 toward the auxiliary sheet 45 and the transfer of bodily fluid from the auxiliary sheet 45 toward the core wrap 40b. Therefore, the diffusion of excrement on the top sheet can be suppressed and the load on the skin can be reduced.

As shown in FIG. 2, the rear end edge 50R of the protruded and recessed structure portion 50 may be positioned on the rear side with respect to a rear end edge 46R of the auxiliary sheet 45 and on the front side with respect to a rear end edge 40aR of the absorbent core 40a. Further, the front end edge 45F of the auxiliary sheet 45 is positioned on the front side with respect to the front end edge 50F of the protruded and recessed structure portion 50, and is positioned on the rear side with respect to the front end edge 40aF of the absorbent core 40a. Therefore, from the front end edge of the absorbent article toward the rear side, the front end edge of the absorbent article, a front end edge 46F of the top sheet 46, the front end edge 40aF of the absorbent core 40a, the front end edge 45F of the auxiliary sheet 45, the front end edge 50F of the protruded and recessed structure portion 50, a rear end edge 45R of the auxiliary sheet 45, the rear end edge 50R of the protruded and recessed structure portion 50, the rear end edge 40aR of the absorbent core 40a, the rear end edge of the absorbent article, and the rear end edge 46R of the top sheet 46 are arranged with spaces in the front-rear direction L.

The absorbent core 40a may include at least a superabsorbent polymer, and the weight of the superabsorbent polymer may be 60% or more and 100% or less with respect to the weight of the absorbent core 40a. Since the weight of the superabsorbent polymer in the absorbent core 40a is relatively high, the absorbent core 40a easily swells when bodily fluid is absorbed, compared with a configuration in which the ratio of pulp in the absorbent core 40a is low. When the absorbent core 40a swells and the absorbent core 40a bulges toward the auxiliary sheet 45, the bottom portion of the auxiliary sheet 45 and the absorbent core 40a become closer to each other, or the absorbent core 40a enters the recessed portion on the back side of the top portion of the auxiliary sheet 45, which makes it possible to enhance the drawability of bodily fluid toward the absorbent core 40a. The weight of the superabsorbent polymer can be calculated based on the water retention amount of the absorbent body. The larger the weight (the higher the ratio) of the superabsorbent polymer, the larger the water retention amount of the entire absorbent body.

Each of the pair of low-basis-weight portions 41 straddles the article front-rear center 10LC and may be curved toward the outer side in the width direction W from the article front-rear center 10LC side toward the front-rear outer side. In the present embodiment, each low-basis-weight portion 41 extends toward the widthwise outer side from the center of the low-basis-weight portion 41 toward the front side in the front-rear direction L, and also extends toward the widthwise outer side from the center of the low-basis-weight portion 41 toward the rear side in the front-rear direction L. The low-basis-weight portion 41 straddles the article front-rear center, and when the absorbent article is interposed between the legs of the wearer, the absorbent core 40a is deformed with the low-basis-weight portion 41 as a base point. The low-basis-weight portion 41 is curved toward the widthwise outer side from the article front-rear center side toward the front-rear outer side, and a deformation base point due to the low-basis-weight portion 41 easily extends along the inner side of the leg. When the low-basis-weight portion 41 extends along the inner side of the leg and the absorbent core 40a is deformed from the low-basis-weight portion 41 as a base point, a cup shape in which a portion on the widthwise outer side with respect to the low-basis-weight portion 41 for the region between the pair of low-basis-weight portions 41 rises up can be embodied. The cup-shaped bottom portion (a region between the pair of low-basis-weight portions 41) makes it possible to diffuse a bodily fluid in the front-rear direction, and absorption of the bodily fluid by the entire absorbent core 40a makes it possible to enhance the transferability of the bodily fluid from the top sheet 46 toward the absorbent core 40a.

As shown in FIG. 1, the absorbent core 40a has a surrounding portion 42 that surrounds the low-basis-weight portion 41 in a plan view. The surrounding portion 42 is a portion arranged so as to surround the low-basis-weight portion 41, and can be set as a portion at 5 mm toward the widthwise outer side from the outer edge of the low-basis-weight portion 41 and 5 mm toward the front-rear outer side from the outer edge of the low-basis-weight portion 41. The basis weight of the superabsorbent polymer in the surrounding portion 42 may be higher than the basis weight of the superabsorbent polymer in regions on the outer side with respect to the low-basis-weight portion 41 and the surrounding portion 42. The region on the outer side with respect to the low-basis-weight portion 41 and the surrounding portion can be set as a portion at 5 mm toward the widthwise outer side from the outer edge of the surrounding portion 42 and 5 mm toward the front-rear outer side from the side edge of the surrounding portion 42. Since the basis weight of the superabsorbent polymer in the surrounding portion 42 that surrounds the low-basis-weight portion 41 is high, the moisture drawn into the low-basis-weight portion 41 can be absorbed by the superabsorbent polymer. The low-basis-weight portion 41 more easily swells, and an effect of suppressing the transfer of excrement from the overlaid region R11 side to the crotch region side can be more easily obtained.

A front end edge 82F of the rear base point portion 82 of the leak-proof gather 80 may be positioned on the rear side with respect to the rear end edge of the protruded and recessed structure portion 50. The contraction portion 83 of the leak-proof gather rises up with the rear base point portion 82 as a fulcrum, and a pocket covered with the leak-proof gather 80 is formed on the front side with respect to the rear base point portion 82. Since the rear end edge of the protruded and recessed structure portion 50 is positioned in the pocket, even when soft feces is diffused into the protruded and recessed structure portion 50, the soft feces can be stored in the pocket. Therefore, soft feces can be prevented from being attached to the skin and the load on the skin can be reduced.

The absorbent core 40a may overlap the front end edge 82F of the rear base point portion 82 and extend toward the rear side with respect to the front end edge 82F of the rear base point portion 82. That is, the front end edge 82F of the rear base point portion 82 may overlap the absorbent core 40a. Since the absorbent core 40a overlaps the front end edge 82F of the rear base point portion 82 and the absorbent core 40a extends toward the rear side with respect to the front end edge 82F of the rear base point portion 82, the rear base point portion 82 is stabilized by the stiffness of the absorbent core 40a. The leak-proof gather 80 easily rises up, and a pocket that stores soft feces can be easily formed. Therefore, the soft feces is more easily stored in the pocket, soft feces can be prevented from being attached to the skin, and the load on the skin can be reduced.

The waist gather 85 may be arranged on the rear side with respect to the rear end edge 40aR of the absorbent core 40a. At least a part of the waist gather may be arranged on the rear side with respect to the rear end edge of the absorbent core 40a. The waist gather may overlap the absorbent core 40a or may be spaced apart from the absorbent core 40a in the front-rear direction. The region located on the rear side with respect to the rear end edge of the absorbent core 40a easily comes into close contact with the body by the waist gather 85. The region on the front side with respect to the waist gather 85 is less likely to come into close contact with the region that overlaps the waist gather 85, and a pocket that bulges toward the body with respect to the waist gather 85 can be formed. When the pocket having the front end edge of the waist gather 85 as a base point is formed on the front side with respect to the waist gather 85, and the pocket having the front end edge 82F of the rear base point portion 82 as a base point is formed on the front side with respect to the rear end edge of the absorbent core 40a, even in case where soft feces is diffused into the protruded and recessed structure portion 50, the soft feces can be stored in the pockets. Therefore, the soft feces is more easily stored in the pocket, soft feces can be prevented from being attached to the skin, and the load on the skin can be reduced.

### (4) Method for Manufacturing Absorbent Article

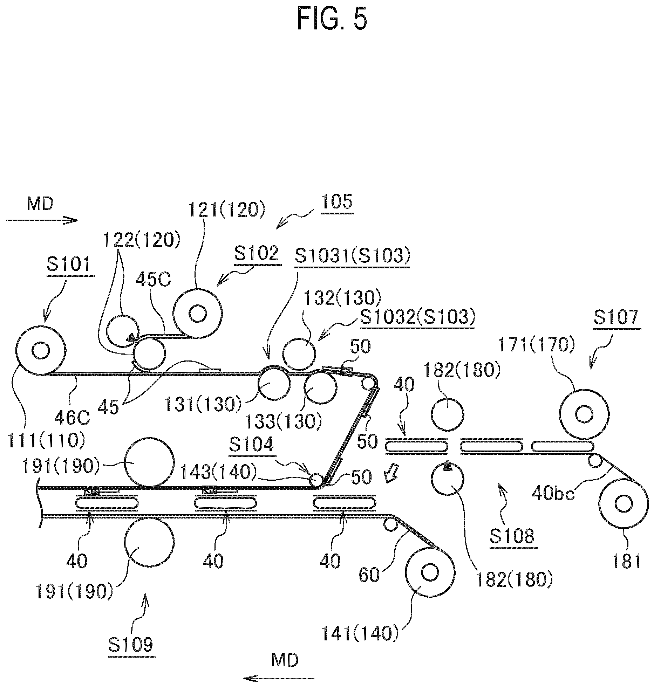

Next, a method for manufacturing an absorbent article will be described with reference to FIG. 5. FIG. 5 is a diagram schematically showing an example of a method for manufacturing an absorbent article and an apparatus for manufacturing an absorbent article. A method S100 for manufacturing an absorbent article may include a top sheet transport step S101, a laminate forming step S102, a shaping step S103, a core molding step S107, a wrapping step S108, an overlaying step S104, and a joining step S109. The apparatus for manufacturing an absorbent article may include a top sheet supply mechanism 110, an auxiliary sheet supply mechanism 120, a shaping mechanism 130, a core molding mechanism 170, a wrapping mechanism 180, an overlaying mechanism 140, and a joining mechanism 190.

The top sheet transport step S101 is performed by the top sheet supply mechanism 110 and transports the top sheet continuous body 46C in which the top sheet that comes into contact with the wearer is continuous. The top sheet supply mechanism 110 includes at least an unwinding roll 111 having the top sheet continuous body 46C to be processed wound thereon in a roll form and configured to unwind the top sheet continuous body 46C toward a transport direction MD.

The laminate forming step S102 is positioned on the downstream side in the transport direction MD with respect to the top sheet transport step S101. The laminate forming step S102 is performed by the auxiliary sheet supply mechanism 120 to overlay the auxiliary sheet 45 on the top sheet continuous body 46C to form a sheet laminate. The auxiliary sheet supply mechanism 120 includes an unwinding roll 121 having an auxiliary sheet continuous body 45C in which the auxiliary sheet 45 is continuous to be processed wound thereon in a roll form and configured to unwind the auxiliary sheet continuous body 45C toward a transport direction MD, and a pair of transport rolls 122 configured to cut the auxiliary sheet continuous body 45C supplied from the unwinding roll 121 by a cutter, and overlay the cut auxiliary sheet 45 on the top sheet continuous body 46C. In the laminate forming step S102, the auxiliary sheets 45 are arranged with spaces in the transport direction MD. It should be noted that since the auxiliary sheets 45 of the present embodiment are arranged with spaces in the front-rear direction L, the auxiliary sheets 45 are intermittently arranged in the transport direction MD. However, in a modified example, in an aspect in which the auxiliary sheet 45 is continuous in the front-rear direction L, the auxiliary sheet continuous body may be overlaid on the top sheet continuous body. Further, in a modified example, in an aspect in which the auxiliary sheet is not provided, it is not necessary to include the laminate forming step S102.

The shaping step S103 is positioned on the downstream side in the transport direction MD with respect to the top sheet transport step S101, and in an aspect having the laminate forming step S102, the shaping step S103 is positioned on the downstream side in the transport direction MD with respect to the laminate forming step S 102. The shaping step S103 is performed by the shaping mechanism 130 to form the protruded and recessed structure portion 50 in the top sheet continuous body 46C or the sheet laminate. In the shaping step S103, the protruded and recessed structure portion 50 is formed with spaces in the front-rear direction L at position where at least a part of the protruded and recessed structure portion corresponds to the rear waist region 30. When the shaping step S103 is provided, an absorbent article 10 having the protruded and recessed structure portion 50 on the top sheet 46 can be obtained. Further, by performing the shaping process on the sheet laminate, an absorbent article in which the protruded and recessed structure portion 50 integrally formed with the top sheet 46 and the auxiliary sheet 45 is located at least in the rear waist region can be manufactured. In the shaping step S103, the protruded and recessed structure portions 50 may be formed with spaces in the transport direction MD. An absorbent article 10 having a region in which the protruded and recessed structure portion 50 is formed and a region in which the protruded and recessed structure portion 50 is not formed can be manufactured.

The core molding step S107 is performed by the core molding mechanism 170 to mold the absorbent core 40a having the low-basis-weight portion 41 in which the basis weight of the absorbent material is lower than the basis weight of the surrounding region. The core molding mechanism 170 includes at least a core molding drum 171. On the outer circumferential surface of the core molding drum 171, a plurality of recessed portions are formed along the outer shape of the absorbent core 40a. The absorbent material is overlaid in each of the recessed portions, and the individual absorbent cores 40a are molded. In the recessed portions of the core molding drum 171, protruding portions corresponding to the low-basis-weight portions 41 are located. In the portion in which the protruding portion is arranged, the absorbent material is not overlaid, and the low-basis-weight portion 41 can be formed.

The wrapping step S108 is positioned on the downstream side in the transport direction MD with respect to the core molding step S107. The wrapping step S108 is performed by the wrapping mechanism 180 and covers the absorbent core 40a with a wrap continuous body 40bC in which the core wrap 40b is continuous. The wrapping mechanism 180 includes at least an unwinding roll 181 that unwinds the wrap continuous body 40bC in the transport direction MD, and a cutting device 182 that cuts the wrap continuous body 40bC. In the wrapping step S108, the skin surface side of the absorbent core 40a and the non-skin surface of the absorbent core 40a are covered with the wrap continuous body 40bC supplied from the unwinding roll 181. Next, the wrap continuous body 40bC can be cut by the cutting device 182 to obtain the individual absorbent bodies 40. It should be noted that, in the wrapping step S108, the wrap continuous body 40bC that covers the skin surface side of the absorbent core 40a and the non-skin surface of the absorbent core 40a may be cut.

The overlaying step S104 is positioned on the downstream side in the transport direction MD with respect to the shaping step S103 and on the downstream side in the transport direction MD with respect to the wrapping step S108. The overlaying step S104 is performed by the overlaying mechanism 140 to overlay the top sheet continuous body or sheet laminate subjected to the shaping process on the absorbent body 40 and the back sheet 60. The overlaying mechanism 140 includes at least an unwinding roll 141 and an absorbent body supply mechanism (not shown). The unwinding roll 141 has a back sheet continuous body 60C in which the back sheet 60 is continuous to be processed wound thereon in a roll form, and unwinds the back sheet continuous body 60C in the transport direction MD. In the absorbent body supply mechanism, the absorbent bodies 40 are arranged with spaces on the back sheet continuous body 60C in the transport direction MD. Next, an absorbent article 10 can be manufactured by cutting the sheet along the outer edge of the absorbent article 10 with a cutting mechanism (not shown). In the overlaying step S104, the top sheet continuous body and the absorbent core 40a are overlaid so that the protruded and recessed structure portion 50 and the low-basis-weight portion 41 overlap in the overlaid region S11 and the low-basis-weight portion 41 is arranged not to overlap the protruded and recessed structure portion 50 in the low-basis-weight region R12. According to the method for manufacturing an absorbent article, an absorbent article having the overlaid region R11 that overlaps the protruded and recessed structure portion 50 and the low-basis-weight portion 41, and the low-basis-weight region R12 that is located on the front side with respect to the front end edge of the protruded and recessed structure portion 50 can be manufactured.

In the laminate forming step S102, the top sheet continuous body 46C and the auxiliary sheet 45 may be joined to each other. A step of joining the top sheet continuous body 46C and the auxiliary sheet 45 may be a step before the shaping step S103. The top sheet continuous body 46C and the auxiliary sheet 45 can be integrally arranged, and problems such as sheet folding in the process of transporting the top sheet continuous body 46C and the auxiliary sheet 45 can be suppressed. It is more suitable that, in the step of joining the top sheet continuous body 46C and the auxiliary sheet 45, the top sheet continuous body 46C and the auxiliary sheet 45 may be joined by thermal welding or ultrasonic welding. According to the above-described configuration, compared with a configuration in which the top sheet continuous body and the auxiliary sheet are joined via a hotmelt adhesive, a decrease in the drawability of bodily fluid due to the adhesive can be suppressed. Therefore, the top sheet 46 and the auxiliary sheet 45 can be integrally formed while suppressing a decrease in the drawability of the bodily fluid, and the transferability of bodily fluid from the top sheet toward the auxiliary sheet 45 can be enhanced.

In addition, the method for manufacturing an absorbent article may further include, after the overlaying step S104, a joining step S109 of joining the non-skin surface of the protruded and recessed structure portion 50 and the non-skin side member that comes into contact with the non-skin surface of the protruded and recessed structure portion 50 in the joining region. The joining step S109 is performed by the joining mechanism 190. The joining mechanism 190 includes at least a pressing device 191. The pressing device 191 has a pair of rolls, and allows a laminate in which the top sheet continuous body (or sheet laminate), the absorbent body, and the back sheet are overlaid to pass between the pair of rolls, and presses the laminate with the pair of rolls. The above-described joining step makes it possible to join the non-skin surface of the protruded and recessed structure portion 50 and the non-skin side member that comes into contact with the non-skin surface of the protruded and recessed structure portion 50. In the present embodiment, the non-skin surface of the protruded and recessed structure portion 50 is the non-skin surface of the auxiliary sheet 45, and in the aspect in which the auxiliary sheet is not provided, the non-skin surface of the top sheet continuous body 46C is used. Further, the non-skin surface member is a member that comes into contact with the non-skin surface of the protruded and recessed structure portions 50, and in the present embodiment, the non-skin surface member is the core wrap 40b. It should be noted that, in an aspect in which the auxiliary sheet 45 is provided and the protruded and recessed structure portion 50 is not formed on the auxiliary sheet, the non-skin surface of the protruded and recessed structure portion 50 may constitute the non-skin surface of the top sheet continuous body, and the non-skin side member may constitute the auxiliary sheet. The joining region is a region in which the non-skin surface of the protruded and recessed structure portion 50 and the non-skin side member are joined in the joining step, and may be the entire region in which the non-skin surface of the protruded and recessed structure portion 50 and the non-skin side member come into contact with each other. In the joining step, at least the joining region may be located in a region in which the low-basis-weight portion 41 is not arranged. The portion in which the protruded and recessed structure portion 50 is formed has a protruded and recessed shape in the thickness direction, and it is difficult to obtain an area in contact with the non-skin surface member compared with a smooth portion. In addition, in the portion in which the low-basis-weight portion 41 is arranged, the thickness of the absorbent core 40a is small compared with the surrounding portion, and it is difficult to obtain an area in contact with portion in which the protruded and recessed structure portion 50 is formed. When the joining region is located only in the portion in which the low-basis-weight portion 41 is arranged and the portion in which the protruded and recessed structure portion 50 is formed, it is difficult to obtain the joining force of the joining region in some cases. However, when at least the joining region in a region in which the low-basis-weight portion 41 is not arranged is located, the joining force of the joining region can be obtained.

The shaping mechanism 130 may include at least a first shaping roll 131, a second shaping roll 132, and a third shaping roll 133. The first shaping roll 131 and the second shaping roll 132 are arranged to face each other, and a first shaping step S1031 is performed in which a first shaping process of shaping a part of the protruded and recessed structure portion 50 is performed on the sheet laminate that passes between the rolls. Further, the second shaping roll 132 and the third shaping roll 133 are arranged to face each other, and a second shaping step S1032 is performed in which a second shaping process of shaping the other part of the protruded and recessed structure portion 50 is performed on the sheet laminate that passes between the rolls. That is, the shaping step S103 may include the first shaping step S1031 and the second shaping step S1032. The second shaping step S1032 is positioned on the downstream side in the transport direction MD with respect to the first shaping step S1031. Since the first shaping step S1031 and the second shaping step S1032 are provided and the shaping processes are performed in a stepwise manner, the protruded and recessed structure portion 50 is easily formed to a detailed part compared with the step of forming the protruded and recessed structure portion at one time. Further, since the first shaping step and the second shaping step are performed using the common second shaping roll, a positional deviation between a part of the protruded and recessed structure portion 50 by the first shaping step S1031 and the other part of the protruded and recessed structure portion 50 by the second shaping step S1032 can be suppressed.

The first shaping roll 131 and the second shaping roll 132 may be formed of a combination of a disk roll and a pin roll, and similarly, the second shaping roll 132 and the third shaping roll 133 may be formed of a combination of a disk roll and a pin roll. More specifically, the disk roll includes protruding ridges arranged with fixed spaces in the roll width direction and a plurality of rows of recessed grooves located between adjacent protruding ridges. The protruding ridges and the recessed grooves are alternately located in the roll width direction, and are located in a manner of continuing in the roll circumferential direction. On the other hand, the pin roll includes a plurality of pins located on the outer circumferential surface so as to engage with the recessed grooves of the disk roll. The pins are arranged with fixed spaces in the roll width direction so as not to come into contact with the protruding ridges, and the pins are arranged substantially linearly along the outer circumferential surface in the roll circumferential direction with fixed spaces. By passing the sheet laminate between the disk roll and the pin roll, the position facing the pin and the recessed groove and the position corresponding to the protruding ridge can be stretched to form the protruded and recessed structure portion 50.

In the first shaping step S1031, the protruded and recessed structure portion 50 may be shaped with spaces in the first direction which is one of the front-rear direction L and the width direction W, and in the second shaping step S1032, the protruded and recessed structure portion 50 may be shaped with spaces in the second direction which is the other of the front-rear direction L and the width direction W. For example, the protruded and recessed structure portion 50 formed by the first shaping step S1031 may have the first top portion 511, the first bottom portion 521, and the first wall portion 531, and the protruded and recessed structure portion 50 formed by the second shaping step S1032 may have the second top portion 512, the second bottom portion 522, and the second wall portion 532. When the protruded and recessed structure portion 50 with spaces in the first direction and the protruded and recessed structure portion 50 with spaces in the second direction are shaped at one time, the material is less likely to stretch at the time of the shaping process. More specifically, when the protruded and recessed structure portion 50 is formed with spaces in the first direction, the material is shaped while extending in the first direction. At the same time, when the protruded and recessed structure portion 50 is formed with spaces in the second direction, the material is less likely to extend in the second direction, and there is a risk that the material may be broken or the protruded and recessed structure portion 50 cannot be appropriately molded. However, since the protruded and recessed structure portion 50 with spaces in the first direction and the protruded and recessed structure portion 50 with spaces in the second direction can be formed in a stepwise manner, the protruded and recessed structure portion 50 can be appropriately molded while preventing damage to the material.

Further, in the shaping step S103, the protruded and recessed structure portion 50 may be formed in a part of the sheet laminate in a crossing direction CD that is orthogonal to the transport direction MD. By forming the protruded and recessed structure portion 50 in a part in the crossing direction CD instead of in the entire region of the sheet laminate in the crossing direction CD, a portion to be stretched and a portion not to be stretched can be located at the time of the shaping process, which makes it possible to stabilize the position of the stretched portion and makes it easier to form the protruded and recessed structure portion 50 in an intended position and shape. Further, when the stretched portion and the non-stretched portion are located at the time of the shaping process, the material can be prevented from being broken due to the stretching of the material at the time of the shaping process.

As described above, although the contents of the present invention were disclosed through the embodiments of the present invention, it should not be understood that the description or drawing constituting any part of this disclosure limits the present invention. From this disclosure, various alternative embodiments, examples and operational techniques will be apparent to those skilled in the art. Therefore, the technical scope of the present invention is defined only by the matters for specifying the invention according to the claims which are reasonable from the above description.

### [Industrial applicability]

According to the present aspect, an absorbent article capable of suppressing the diffusion of excrement such as soft feces on the top sheet and reducing the load on the skin can be provided.

### REFERENCE SIGNS LIST

10: disposable diaper, 20: front waist region, 25: crotch region, 30: rear waist region, 40: absorbent body, 41: low-basis-weight portion, 42: surrounding portion, 45: auxiliary sheet, 46: top sheet, 46C: top sheet continuous body, 50: protruded and recessed structure portion, 51: top portion, 52: bottom portion, 53: wall portion, 60: rear sheet, R11: overlaid region, R12: low-basis-weight region, R13: protruded and recessed region, R14: non-overlaid region, S101: top sheet transport step, S102: laminate forming step, S103: shaping step, S1031: first shaping step, S1032: second shaping step, S104: overlaying step, S107: core molding step, S108: wrapping step, S109: joining step, L: front-rear direction, T: thickness direction, T1: skin facing surface side, T2: non-skin facing surface side, W: width direction

## Claims

1. An absorbent article (10) having a front waist region (20) , a rear waist region (30), and a crotch region (25) that is positioned between the front waist region (20) and the rear waist region (30), and having a front-rear direction (L) that is directed from the front waist region (20) toward the rear waist region (30), and a width direction (W) that is orthogonal to the front-rear direction, the absorbent article comprising:
an absorbent core (40a) that straddles the crotch region, extends toward at least any one of the front waist region and the rear waist region, and has at least a superabsorbent polymer; and
a top sheet (46) that is positioned on a skin facing surface side (T1) with respect to the absorbent core and comes into contact with a wearer, wherein
in at least the rear waist region of the top sheet, a protruded and recessed structure portion (50) having a top portion (51) that protrudes toward the skin facing surface side (T1), a bottom portion (52) that protrudes toward a non-skin facing surface side (T2) , and a wall portion (53) that connects the top portion and the bottom portion is located,
the absorbent core has a low-basis-weight portion (41) in which a basis weight of an absorbent material is lower than a basis weight of a surrounding region,
a front end edge (50F) of the protruded and recessed structure portion is positioned on a rear side with respect to an article front-rear center (10LC), which is a center of the absorbent article in the front-rear direction,
in a core arrangement region in which the absorbent core is arranged, an overlaid region (R11) in which the protruded and recessed structure portion (50) and the low-basis-weight portion (41) overlap, and a low-basis-weight region (R12) that overlaps the low-basis-weight portion 41 in a plan view and in which the protruded and recessed structure portion 50 is not arranged, and
at least a part of the low-basis-weight region (R12) is arranged on a front side with respect to the protruded and recessed structure portion (50).

2. The absorbent article according to claim 1, further comprising:
a core wrap (40b) that covers a skin surface and a non-skin surface of the absorbent core (40a), wherein
the low-basis-weight portion (41) is a slit in which the absorbent material is not substantially arranged, and
the core wrap (40b) positioned on a skin surface side of the absorbent core (40a) and the core wrap (40b) positioned on the non-skin surface of the absorbent core (40a) are joined in a region that overlaps the slit.

3. The absorbent article according to claim 1 or 2, wherein
in the core arrangement region, a protruded and recessed region (R13) in which the protruded and recessed structure portion (50) is arranged and the low-basis-weight portion (41) is not arranged,
at least a part of the protruded and recessed region (R13) is arranged on the rear side with respect to a rear end edge of the low-basis-weight portion (41), and
a front-rear length of the protruded and recessed region (R13) arranged on the rear side with respect to the rear end edge of the low-basis-weight portion (41) is longer than a front-rear length of the overlaid region (R11).

4. The absorbent article according to any one of claims 1 to 3, further comprising:
an auxiliary sheet (45) that comes into contact with a non-skin facing surface of the top sheet (46) and has a higher density than the top sheet (46), wherein
the auxiliary sheet (45) is arranged to straddle the overlaid region (R11) and the low-basis-weight region (R12) arranged on the front side with respect to the protruded and recessed structure portion (50).

5. The absorbent article according to claim 4, further comprising:
the core wrap (40b) that covers the absorbent core (40a) and comes into contact with a non-skin surface of the auxiliary sheet (45), wherein
a density of the core wrap (40b) is higher than a density of the auxiliary sheet (45).

6. The absorbent article according to any one of claims 1 to 5, wherein
a pair of low-basis-weight portions (41) are located with a widthwise center of the absorbent article interposed therebetween, and
each of the pair of low-basis-weight portions (41) straddles the article front-rear center and is curved toward a widthwise outer side from the article front-rear center side toward a front-rear outer side.

7. The absorbent article according to any one of claims 1 to 6, wherein,
in the core arrangement region, a protruded and recessed region (R13) in which the protruded and recessed structure portion (50) is arranged and the low-basis-weight portion (41) is not arranged, and a non-overlaid region (R14) in which the protruded and recessed structure portion (50) is not arranged and the low-basis-weight portion (41) is not arranged,
the non-overlaid region (R14) has a front non-overlaid region (R14F) that extends toward a front side from the front end edge of the protruded and recessed structure portion (50), and
a basis weight of the superabsorbent polymer in the protruded and recessed region (R13) is higher than a basis weight of the superabsorbent polymer in the front non-overlaid region (R14F).

8. The absorbent article according to any one of claims 1 to 7, wherein
the absorbent core has a surrounding portion (42) that surrounds the low-basis-weight portion (41) in a plan view, and
a basis weight of the superabsorbent polymer in the surrounding portion (42) is higher than a basis weight of the superabsorbent polymer in a region on an outer side with respect to the low-basis-weight portion (41) and the surrounding portion (42).

9. The absorbent article according to any one of claims 1 to 8, further comprising:
a rising leak-proof gather (80) having a contraction portion that contracts by contraction of a leak-proof elastic member (71), and a rear base point portion (82) that serves as a rising fulcrum of the contraction portion on the rear side with respect to the contraction portion, wherein
a front end edge (82F) of the rear base point portion (82) is positioned on the rear side with respect to a rear end edge of the protruded and recessed structure portion (50).

10. The absorbent article according to claim 9, wherein
the absorbent core (40a) overlaps the front end edge (82F) of the rear base point portion (82) and extends toward the rear side with respect to the front end edge of the rear base point portion (82).

11. The absorbent article according to claim 10, further comprising:
a waist gather (85) that stretches and contracts in the width direction in the rear waist region (30), wherein
the waist gather is arranged on the rear side with respect to a rear end edge of the absorbent core (40a).

12. A method for manufacturing an absorbent article (10) having a front waist region (20), a rear waist region (30), and a crotch region (25) that is positioned between the front waist region (20) and the rear waist region (30), and having a front-rear direction (L) that is directed from the front waist region (20) toward the rear waist region (30), and a width direction (W) that is orthogonal to the front-rear direction (L), the method comprising:
a core molding step (S107) of molding an absorbent core (40a) having a low-basis-weight portion (41) in which a basis weight of an absorbent material is lower than a basis weight of a surrounding region;
a top sheet transport step (S101) of transporting a top sheet continuous body in which a top sheet (46) that comes into contact with a wearer is continuous;
a shaping step (S 103)of forming a protruded and recessed structure portion (50) having a top portion (51) that protrudes toward a skin facing surface side (T1), a bottom portion (52) that protrudes toward a non-skin facing surface side (T2), and a wall portion (53) that connects the top portion (51) and the bottom portion (52) at least in the top sheet continuous body; and
an overlaying step (S104) of overlaying the top sheet continuous body and the absorbent core (40a) in a manner such that the protruded and recessed structure portion (50) and the low-basis-weight portion (41) overlap in an overlaid region (R11), and the low-basis-weight portion (41) is arranged not to overlap the protruded and recessed structure portion (50) in a low-basis-weight region (R12), wherein
in the overlaying step, the low-basis-weight region (R12) is located on a front side with respect to the protruded and recessed structure portion (50).

13. The method for manufacturing an absorbent article according to claim 12, further comprising:
a joining step (S109) of joining a non-skin surface of the protruded and recessed structure portion (50) and a non-skin side member that comes into contact with the non-skin surface of the protruded and recessed structure portion (50) in a joining region, wherein
in the joining step, at least the joining region is located in a region in which the low-basis-weight portion is not arranged.

## Patentansprüche

1. Absorbierender Artikel (10), der Folgendes aufweist: einen vorderen Taillenbereich (20), einen hinteren Taillenbereich (30) und einen Schrittbereich (25), der zwischen dem vorderen Taillenbereich (20) und dem hinteren Taillenbereich (30) positioniert ist, und der Folgendes aufweist: eine Vorn-Hinten-Richtung (L), die von dem vorderen Taillenbereich (20) zu dem hinteren Taillenbereich (30) gerichtet ist, und eine Breitenrichtung (W), die rechtwinklig zu der Vorn-Hinten-Richtung vorliegt, wobei der absorbierende Artikel Folgendes umfasst:
einen Saugkern (40a), der den Schrittbereich überspannt, sich zu mindestens einem des vorderen Taillenbereichs und des hinteren Taillenbereichs erstreckt und mindestens ein superabsorbierendes Polymer aufweist; und
eine obere Lage (46), die auf einer der Haut zugewandten Oberflächenseite (T1) in Bezug auf den Saugkern positioniert ist und mit einem Träger in Kontakt kommt, wobei
sich in zumindest dem hinteren Taillenbereich der oberen Lage ein vorstehender und vertiefter Strukturteil (50) befindet, der Folgendes aufweist: einen oberen Teil (51), der zu der der Haut zugewandten Oberflächenseite (T1) vorsteht, einen unteren Teil (52), der zu einer nicht der Haut zugewandten Oberflächenseite (T2) vorsteht, und einen Wandteil (53), der den oberen Teil und den unteren Teil verbindet,
der Saugkern einen Teil mit geringem Flächengewicht (41) aufweist, in dem ein Flächengewicht eines absorbierenden Materials geringer ist als ein Flächengewicht eines umgebenden Bereichs,
ein vorderer Endrand (50F) des vorstehenden und vertieften Strukturteils auf einer hinteren Seite in Bezug auf eine Vorn-Hinten-Mitte des Artikels (10LC), die eine Mitte des absorbierenden Artikels in der Vorn-Hinten-Richtung ist, positioniert ist,
in einem Kernanordnungsbereich, in dem der Saugkern angeordnet ist, ein überlagerter Bereich (R11), in dem der vorstehende und vertiefte Strukturteil (50) und der Teil mit geringem Flächengewicht (41) überlappen, und ein Bereich mit geringem Flächengewicht (R12), der den Teil mit geringem Flächengewicht 41 in einer Draufsicht überlappt und in dem der vorstehende und vertiefte Strukturteil 50 nicht angeordnet ist, und
zumindest ein Teil des Bereichs mit geringem Flächengewicht (R12) auf einer vorderen Seite in Bezug auf den vorstehenden und vertieften Strukturteil (50) angeordnet ist.

2. Absorbierender Artikel nach Anspruch 1, der weiter Folgendes umfasst:
eine Kernumhüllung (40b), die eine Hautoberfläche und eine Nicht-Hautoberfläche des Saugkerns (40a) bedeckt, wobei
der Teil mit geringem Flächengewicht (41) ein Spalt ist, in dem das absorbierende Material nicht erheblich angeordnet ist, und
die Kernumhüllung (40b), die auf einer Hautoberflächenseite des Saugkerns (40a) positioniert ist, und die Kernumhüllung (40b), die auf der Nicht-Hautoberfläche des Saugkerns (40a) positioniert ist, in einem Bereich, der den Spalt überlappt, verbunden sind.

3. Absorbierender Artikel nach Anspruch 1 oder 2, wobei
in dem Kernanordnungsbereich ein vorstehender und vertiefter Bereich (R13), in dem der vorstehende und vertiefte Strukturteil (50) angeordnet ist und der Teil mit geringem Flächengewicht (41) nicht angeordnet ist,
zumindest ein Teil des vorstehenden und vertieften Bereichs (R13) auf der hinteren Seite in Bezug auf einen hinteren Endrand des Teils mit geringem Flächengewicht (41) angeordnet ist, und
eine Vorn-Hinten-Länge des vorstehenden und vertieften Bereichs (R13), die auf der hinteren Seite in Bezug auf den hinteren Endrand des Teils mit geringem Flächengewicht (41) angeordnet ist, länger ist als eine Vorn-Hinten-Länge des überlagerten Bereichs (R11).

4. Absorbierender Artikel nach einem der Ansprüche 1 bis 3, der weiter Folgendes umfasst:
eine Hilfslage (45), die mit einer nicht der Haut zugewandten Oberfläche der oberen Lage (46) in Kontakt kommt und eine höhere Dichte aufweist als die obere Lage (46), wobei
die Hilfslage (45) zum Überspannen des überlagerten Bereichs (R11) und des Bereichs mit geringem Flächengewicht (R12), der auf der vorderen Seite in Bezug auf den vorstehenden und vertieften Strukturteil (50) angeordnet ist, angeordnet ist.

5. Absorbierender Artikel nach Anspruch 4, der weiter Folgendes umfasst:
die Kernumhüllung (40b), die den Saugkern (40a) bedeckt und mit einer Nicht-Hautoberfläche der Hilfslage (45) in Kontakt kommt, wobei
eine Dichte der Kernumhüllung (40b) höher ist als eine Dichte der Hilfslage (45).

6. Absorbierender Artikel nach einem der Ansprüche 1 bis 5, wobei
sich ein Paar von Teilen mit geringem Flächengewicht (41) mit einer dazwischen eingefügten Mitte in der Breite des absorbierenden Artikels befinden und
jeder des Paars von Teilen mit geringem Flächengewicht (41) die Vorn-Hinten-Mitte des Artikels überspannt und in Richtung einer Außenseite in der Breite von der Vorn-Hinten-Mittelseite des Artikels in Richtung einer Vorn-Hinten-Außenseite gekrümmt ist.

7. Absorbierender Artikel nach einem der Ansprüche 1 bis 6, wobei
in dem Kernanordnungsbereich ein vorstehender und vertiefter Bereich (R13), in dem der vorstehende und vertiefte Strukturteil (50) angeordnet ist und der Teil mit geringem Flächengewicht (41) nicht angeordnet ist, und ein nicht-überlagerter Bereich (R14), in dem der vorstehende und vertiefte Strukturteil (50) nicht angeordnet ist und der Teil mit geringem Flächengewicht (41) nicht angeordnet ist,
der nicht-überlagerte Bereich (R14) einen vorderen nicht-überlagerten Bereich (R14F) aufweist, der sich in Richtung einer vorderen Seite von dem vorderen Endrand des vorstehenden und vertieften Strukturteils (50) erstreckt, und
ein Flächengewicht des superabsorbierenden Polymers in dem vorstehenden und vertieften Bereich (R13) höher ist als ein Flächengewicht des superabsorbierenden Polymers in dem vorderen nicht-überlagerten Bereich (R14F).

8. Absorbierender Artikel nach einem der Ansprüche 1 bis 7, wobei
der Saugkern einen umgebenden Teil (42) aufweist, der den Teil mit geringem Flächengewicht (41) in einer Draufsicht umgibt, und
ein Flächengewicht des superabsorbierenden Polymers in dem umgebenden Teil (42) höher ist als ein Flächengewicht des superabsorbierenden Polymers in einem Bereich auf einer Außenseite in Bezug auf den Teil mit geringem Flächengewicht (41) und den umgebenden Teil (42).

9. Absorbierender Artikel nach einem der Ansprüche 1 bis 8, der weiter Folgendes umfasst:
eine aufrichtende auslaufsichere Raffung (80), die Folgendes aufweist: einen zusammenziehenden Teil, der sich durch Zusammenziehen eines auslaufsicheren elastischen Elements (71) zusammenzieht, und einen hinteren Basispunktteil (82), der als ein aufrichtender Stützpunkt des zusammenziehenden Teils auf der hinteren Seite in Bezug auf den zusammenziehenden Teil dient, wobei
ein vorderer Endrand (82F) des hinteren Basispunktteils (82) auf der hinteren Seite in Bezug auf einen hinteren Endrand des vorstehenden und vertieften Strukturteils (50) positioniert ist.

10. Absorbierender Artikel nach Anspruch 9, wobei
der Saugkern (40a) den vorderen Endrand (82F) des hinteren Basispunktteils (82) überlappt und sich in Richtung der hinteren Seite in Bezug auf den vorderen Endrand des hinteren Basispunktteils (82) erstreckt.

11. Absorbierender Artikel nach Anspruch 10, der weiter Folgendes umfasst:
eine Taillenraffung (85), die sich in der Breitenrichtung in dem hinteren Taillenbereich (30) dehnt und zusammenzieht, wobei
die Taillenraffung auf der hinteren Seite in Bezug auf einen hinteren Endrand des Saugkerns (40a) angeordnet ist.

12. Verfahren zur Herstellung eines absorbierenden Artikels (10), der Folgendes aufweist: einen vorderen Taillenbereich (20), einen hinteren Taillenbereich (30) und einen Schrittbereich (25), der zwischen dem vorderen Taillenbereich (20) und dem hinteren Taillenbereich (30) positioniert ist, und der Folgendes aufweist: eine Vorn-Hinten-Richtung (L), die von dem vorderen Taillenbereich (20) zu dem hinteren Taillenbereich (30) gerichtet ist, und eine Breitenrichtung (W), die rechtwinklig zu der Vorn-Hinten-Richtung (L) vorliegt, wobei das Verfahren Folgendes umfasst:
einen Kernformgebungsschritt (S107) des Formgebens eines Saugkerns (40a), der einen Teil mit geringem Flächengewicht (41) aufweist, in dem ein Flächengewicht eines absorbierenden Materials geringer ist als ein Flächengewicht eines umgebenden Bereichs;
einen Transportschritt der oberen Lage (S101) des Transportierens eines fortlaufenden Körpers der oberen Lage, in dem eine obere Lage (46), die mit einem Träger in Kontakt kommen, fortlaufend ist;
einen Formungsschritt (S103) des Ausbildens eines vorstehenden und vertieften Strukturteils (50), der Folgendes aufweist: einen oberen Teil (51), der zu einer der Haut zugewandten Oberflächenseite (T1) vorsteht, einen unteren Teil (52), der zu einer nicht der Haut zugewandten Oberflächenseite (T2) vorsteht, und einen Wandteil (53), der den oberen Teil (51) und den unteren Teil (52) zumindest in dem fortlaufenden Körper der oberen Lage verbindet; und
einen Überlagerungsschritt (S104) des Überlagerns des fortlaufenden Körpers der oberen Lage und des Saugkerns (40a) in einer derartigen Weise, dass der vorstehende und vertiefte Strukturteil (50) und der Teil mit geringem Flächengewicht (41) in einem überlagerten Bereich (R11) überlappen und der Teil mit geringem Flächengewicht (41) angeordnet ist, um mit dem vorstehenden und vertieften Strukturteil (50) in dem Bereich mit geringem Flächengewicht (R12) nicht zu überlappen, wobei
in dem Überlagerungsschritt sich der Bereich mit geringem Flächengewicht (R12) auf einer vorderen Seite in Bezug auf den vorstehenden und vertieften Strukturteil (50) befindet.

13. Verfahren zur Herstellung eines absorbierenden Artikels nach Anspruch 12, das weiter Folgendes umfasst:
einen Verbindungsschritt (S109) des Verbindens einer Nicht-Hautoberfläche des vorstehenden und vertieften Strukturteils (50) und eines Nicht-Hautseitenelements, das mit der Nicht-Hautoberfläche des vorstehenden und vertieften Strukturteils (50) in Kontakt kommt, in einem Verbindungsbereich, wobei
in dem Verbindungsschritt sich zumindest der Verbindungsbereich in einem Bereich befindet, in dem der Teil mit geringem Flächengewicht nicht angeordnet ist.

## Revendications

1. Article absorbant (10) ayant une région avant au niveau de la taille (20), une région arrière au niveau de la taille (30), et une région au niveau de l'entrejambe (25) qui est positionnée entre la région avant au niveau de la taille (20) et la région arrière au niveau de la taille (30), et ayant une direction allant dans le sens avant-arrière (L) qui est dirigée depuis la région avant au niveau de la taille (20) vers la région arrière au niveau de la taille (30), et une direction allant dans le sens de la largeur (W) qui est orthogonale par rapport à la direction allant dans le sens avant-arrière, l'article absorbant comportant :
une partie centrale absorbante (40a) qui chevauche la région au niveau de l'entrejambe, s'étend vers au moins l'une quelconque parmi la région avant au niveau de la taille et la région arrière au niveau de la taille, et a au moins un polymère superabsorbant ; et
une feuille supérieure (46) qui est positionnée sur un côté de surface orientée vers la peau (T1) par rapport à la partie centrale absorbante et qui vient se mettre en contact avec un utilisateur, dans lequel
dans au moins la région arrière au niveau de la taille de la feuille supérieure se trouve une partie formant structure saillante et évidée (50) ayant une partie supérieure (51) qui fait saillie vers le côté de surface orientée vers la peau (T1), une partie inférieure (52) qui fait saillie vers un côté de surface non orientée vers la peau (T2), et une partie formant paroi (53) qui relie la partie supérieure et la partie inférieure,
la partie centrale absorbante a une partie de faible masse surfacique (41) dans laquelle une masse surfacique d'un matériau absorbant est inférieure à une masse surfacique d'une région environnante,
un bord d'extrémité avant (50F) de la partie formant structure saillante et évidée est positionné sur un côté arrière par rapport à un centre allant dans le sens avant-arrière de l'article (10LC), qui est un centre de l'article absorbant dans la direction allant dans le sens avant-arrière,
dans une région d'agencement de la partie centrale dans laquelle la partie centrale absorbante est agencée, une région superposée (R11) dans laquelle la partie formant structure saillante et évidée (50) et la partie de faible masse surfacique (41) se chevauchent, et une région de faible masse surfacique (R12) qui chevauche la partie de structure de faible masse surfacique 41 dans une vue en plan et dans laquelle la partie formant structure saillante et évidée 50 n'est pas agencée, et
au moins une partie de la région de faible masse surfacique (R12) est agencée sur un côté avant par rapport à la partie formant structure saillante et évidée (50).

2. Article absorbant selon la revendication 1, comportant par ailleurs :
une feuille de revêtement de partie centrale (40b) qui recouvre une surface orientée vers la peau et une surface non orientée vers la peau de la partie centrale absorbante (40a), dans lequel
la partie de faible masse surfacique (41) est une fente dans laquelle le matériau absorbant n'est pas sensiblement agencé, et
la feuille de revêtement de partie centrale (40b) positionnée sur un côté de surface orientée vers la peau de la partie centrale absorbante (40a) et la feuille de revêtement de partie centrale (40b) positionnée sur la surface non orientée vers la peau de la partie centrale absorbante (40a) sont assemblées dans une région qui chevauche la fente.

3. Article absorbant selon la revendication 1 ou la revendication 2, dans lequel
dans la région d'agencement de la partie centrale, une région saillante et évidée (R13) dans laquelle la partie formant structure saillante et évidée (50) est agencée et dans laquelle la partie de faible masse surfacique (41) n'est pas agencée,
au moins une partie de la région saillante et évidée (R13) est agencée sur le côté arrière par rapport à un bord d'extrémité arrière de la partie de faible masse surfacique (41), et
une longueur allant dans le sens avant-arrière de la région saillante et évidée (R13) agencée sur le côté arrière par rapport au bord d'extrémité arrière de la partie de faible masse surfacique (41) est plus longue qu'une longueur allant dans le sens avant-arrière de la région superposée (R11).

4. Article absorbant selon l'une quelconque des revendications 1 à 3, comportant par ailleurs :
une feuille auxiliaire (45) qui vient se mettre en contact avec une surface non orientée vers la peau de la feuille supérieure (46) et qui a une densité supérieure à celle de la feuille supérieure (46), dans lequel
la feuille auxiliaire (45) est agencée de manière à chevaucher la région superposée (R11) et la région de faible masse surfacique (R12) agencée sur le côté avant par rapport à la partie formant structure saillante et évidée (50).

5. Article absorbant selon la revendication 4, comportant par ailleurs :
la feuille de revêtement de partie centrale (40b) qui recouvre la partie centrale absorbante (40a) et qui vient se mettre en contact avec une surface non orientée vers la peau de la feuille auxiliaire (45), dans lequel
une densité de la feuille de revêtement de partie centrale (40b) est supérieure à une densité de la feuille auxiliaire (45).

6. Article absorbant selon l'une quelconque des revendications 1 à 5, dans lequel
les parties d'une paire de parties de faible masse surfacique (41) sont placées avec un centre allant dans le sens de la largeur de l'article absorbant positionné entre elles, et
chaque partie de la paire de parties de faible masse surfacique (41) chevauche le centre allant dans le sens avant-arrière de l'article et est incurvée vers un côté extérieur allant dans le sens de la largeur depuis le côté central allant dans le sens avant-arrière de l'article vers un côté extérieur allant dans le sens avant-arrière.

7. Article absorbant selon l'une quelconque des revendications 1 à 6, dans lequel,
dans la région d'agencement de la partie centrale, une région saillante et évidée (R13) dans laquelle la partie formant structure saillante et évidée (50) est agencée et dans laquelle la partie de faible masse surfacique (41) n'est pas agencée, et une région non superposée (R14) dans laquelle la partie formant structure saillante et évidée (50) n'est pas agencée et dans laquelle la partie de faible masse surfacique (41) n'est pas agencée,
la région non superposée (R14) a une région avant non superposée (R14F) qui s'étend vers un côté avant depuis le bord d'extrémité avant de la partie formant structure saillante et évidée (50), et
une masse surfacique du polymère superabsorbant dans la région saillante et évidée (R13) est supérieure à une masse surfacique du polymère superabsorbant dans la région avant non superposée (R14F).

8. Article absorbant selon l'une quelconque des revendications 1 à 7, dans lequel
la partie centrale absorbante a une partie environnante (42) qui entoure la partie de faible masse surfacique (41) dans une vue en plan, et
une masse surfacique du polymère superabsorbant dans la partie environnante (42) est supérieure à une masse surfacique du polymère superabsorbant dans une région sur un côté extérieur par rapport à la partie de faible masse surfacique (41) et à la partie environnante (42).

9. Article absorbant selon l'une quelconque des revendications 1 à 8, comportant par ailleurs :
une fronce anti-fuite montant vers le haut (80) ayant une partie de contraction qui se contracte par contraction d'un élément élastique anti-fuite (71), et une partie de point de base arrière (82) qui sert de point d'appui montant vers le haut de la partie de contraction sur le côté arrière par rapport à la partie de contraction, dans lequel
un bord d'extrémité avant (82F) de la partie de point de base arrière (82) est positionné sur le côté arrière par rapport à un bord d'extrémité arrière de la partie formant structure saillante et évidée (50).

10. Article absorbant selon la revendication 9, dans lequel
la partie centrale absorbante (40a) chevauche le bord d'extrémité avant (82F) de la partie de point de base arrière (82) et s'étend vers le côté arrière par rapport au bord d'extrémité avant de la partie de point de base arrière (82).

11. Article absorbant selon la revendication 10, comportant par ailleurs :
une fronce au niveau de la taille (85) qui s'étire et se contracte dans la direction allant dans le sens de la largeur dans la région arrière au niveau de la taille (30), dans lequel
la fronce au niveau de taille est agencée sur le côté arrière par rapport à un bord d'extrémité arrière de la partie centrale absorbante (40a).

12. Procédé de fabrication d'un article absorbant (10) ayant une région avant au niveau de la taille (20), une région arrière au niveau de la taille (30), et une région au niveau de l'entrejambe (25) qui est positionnée entre la région avant au niveau de la taille (20) et la région arrière au niveau de la taille (30), et ayant une direction allant dans le sens avant-arrière (L) qui est dirigée depuis la région avant au niveau de la taille (20) vers la région arrière au niveau de la taille (30), et une direction allant dans le sens de la largeur (W) qui est orthogonale par rapport à la direction allant dans le sens avant-arrière (L), le procédé comportant :
une étape de moulage de partie centrale (S107) consistant à mouler une partie centrale absorbante (40a) ayant une partie de faible masse surfacique (41) dans laquelle une masse surfacique d'un matériau absorbant est inférieure à une masse surfacique d'une région environnante ;
une étape de transport de feuille supérieure (S101) consistant à transporter un corps continu de feuille supérieure dans lequel une feuille supérieure (46) qui vient se mettre en contact avec un utilisateur est une feuille continue ;
une étape de mise en forme (S103) consistant à mettre en forme une partie formant structure saillante et évidée (50) ayant une partie supérieure (51) qui fait saillie vers un côté de surface orientée vers la peau (T1), une partie inférieure (52) qui fait saillie vers un côté de surface non orientée vers la peau (T2), et une partie formant paroi (53) qui relie la partie supérieure (51) et la partie inférieure (52) au moins dans le corps continu de feuille supérieure ; et
une étape de superposition (S104) consistant à superposer le corps continu de feuille supérieure et la partie centrale absorbante (40a) de telle sorte que la partie formant structure saillante et évidée (50) et la partie de faible masse surfacique (41) se chevauchent dans une région superposée (R11), et la partie de faible masse surfacique (41) est agencée pour ne pas chevaucher la partie formant structure saillante et évidée (50) dans une région de faible masse surfacique (R12), dans lequel
au cours de l'étape de superposition, la région de faible masse surfacique (R12) est située sur un côté avant par rapport à la partie formant structure saillante et évidée (50).

13. Procédé de fabrication d'un article absorbant selon la revendication 12, comportant par ailleurs :
une étape d'assemblage (S109) consistant à assembler une surface non orientée vers la peau de la partie formant structure saillante et évidée (50) et un élément latéral non orienté vers la peau qui vient se mettre en contact avec la surface non orientée vers la peau de la partie formant structure saillante et évidée (50) dans une région d'assemblage, dans lequel
au cours de l'étape d'assemblage, au moins la région d'assemblage est située dans une région dans laquelle la partie de faible masse surfacique n'est pas agencée.
